# EUROPEAN PATENT APPLICATION

(11) **EP 3 770 245 A1**
(43) Date of publication of application: **27.01.2021**
(21) Application number: 20190032.1
(22) Date of filing: 25.01.2017
(51) Int. Cl.: C12M 1/00, C12M 1/10, C12M 3/04, C12M 1/26, C12M 1/33, C12N 5/07

(54) **LARGE-SCALE CELL CULTURE SYSTEM**

(30) Priority: 27.04.2016 JP 2016089837; 19.01.2017 JP 2017007993
(62) Divisional of application: 17788970.6
(71) Applicant: JTEC Corporation, Ibaraki-shi Osaka 567-0086 (JP)
(72) Inventor: Tsumura, Takashi, Osaka 567-0086 (JP); Uemura, Toshimasa, Osaka 567-0086 (JP); Uchita, Yudai, Osaka 567-0086 (JP)
(74) Representative: Vossius & Partner Patentanwälte Rechtsanwälte mbB

(57) **Abstract**

The present invention discloses a system, comprising vessels, wherein the vessels each comprise an outer cylinder part, each of the vessels having a syringe structure in which a head of the vessel, serving as a port for a solution is provided at a distal end of the outer cylinder part, wherein a space in the vessel, closed by a gasket of a plunger is filled with a cell liquid obtained by suspending cells in a culture medium; a connection tool having a filter built therein, wherein the filter is configured to disrupt cell aggregates in the cell liquid into small cell aggregates as a result of the cell aggregates passing through the filter when the cell liquid is transferred from one vessel to the other vessel; and an inter-vessel cell liquid transfer device capable of transferring the cell liquid from one vessel to another vessel, wherein the inter-vessel cell liquid transfer device is capable of holding the outer cylinder parts of the vessels in a state where the heads of the vessels are connected to each other through the connection tool and capable of performing driving so as to push and pull the plunger of one of the vessels.

## Description

### TECHNICAL FIELD

The present invention relates to a large-scale cell culture system suitable for performing large-scale culture of pluripotent stem cells or adhesive cells to be used in regenerative medicine or the like, an inter-vessel cell liquid transfer device used therein, and a rotary cell culture device.

### BACKGROUND ART

Discovery of induced pluripotent stem cells (herein, often referred to as "iPS cells" (Non-Patent Literature 1 to 3) has increased the momentum for practical use of regenerative medicine using such induced pluripotent stem cells. The number of iPS cells in the order of 10⁶, a quantity commonly used in a laboratory, is far from sufficient for using iPS cells in regenerative medicine or the like, and is required to be in the order of 10⁹ to 10¹⁰ for clinical application. However, techniques for large-scale culture thereof have not been fully established. To culture iPS cells with the undifferentiated state thereof maintained, it is generally considered to be necessary to culture the iPS cells on feeder cells such as primary cultures of mouse embryonic fibroblast (MEF) and STO cells. However, contamination with a feeder cell constitutes a significant obstacle to use of iPS cells in regenerative medicine.

In view of this, studies on feeder-free cell culture methods have been conducted, and methods enabling culture without any feeder cell have been developed, such as a method of culturing iPS cells on the surface of a base material coated with Matrigel and a culture method utilizing a coating with laminin or a partial peptide of laminin. In addition, bag culture has been performed in place of dish culture which is commonly conducted. However, it is necessary to repeat culture on a base material with a coating even in a feeder-free culture system, and thus the culture process is complicated and the cost of culture significantly increases, which causes a serious problem of huge cost for treating one patient. On the other hand, in the case of adhesive cells, it is necessary to add an agent after culture to detach adherent cells, and thus, when adhesive cells are to be used in regenerative medicine, contamination with an agent likewise becomes a problem.

To construct a three-dimensional tissue from cells, it is typically necessary to perform three-dimensional culture using an appropriate scaffold material, or to perform spinner culture. However, conventional spinner culture applies a strong mechanical stimulus and causes significant damage to cells, and thus, it is difficult to obtain a large tissue, and even if a large tissue is obtained, the inside often undergoes necrosis. As a countermeasure against this, there exists a series of bioreactors designed to optimize the weight. An RWV (Rotating Wall Vessel) bioreactor, one of such bioreactors, is a rotary bioreactor having a gas exchange function developed by NASA. The present inventors have conducted research and development of, for example, a technique of cartilage regeneration from bone marrow cells, etc., by three-dimensional culture using this RWV bioreactor (Patent Literature 1 to 3).

The present inventors have also developed a rotary cell culture device, in parallel with development of a culture method. For example, Patent Literature 4 to 6 propose devices in which: a flat cylindrical culture vessel having a gas permeable membrane on the back side (rear side) is attached to a horizontal rotation shaft of a rotation control device, and is rotated while being cantilevered at the back face side; the gravity, the buoyancy of spheroids, and the force applied from the flow of the culture medium caused by the rotation are balanced in the culture vessel; a pseudo-micro gravity environment having one-hundredth the gravity of earth by time average is created; and accordingly, a state where the spheroids do not sediment but float in a certain area is realized. In these devices, an observation window is provided on the front side of the culture vessel, images of spheroids are taken by a camera through the window, and accordingly, the rotation speed is controlled in accordance with the growth and the suspended state of cells such that the spheroids are always kept in a suspended state in the certain area.

In such circumstances, the present inventors have proposed a method for performing efficient and large-scale culture of stable iPS cells with undifferentiation property thereof maintained (Patent Literature 7). That is, the invention described in Patent Literature 7 is advantageous in that: since pluripotent stem cells, in particular, iPS cells, are cultured in a pseudo-microgravity environment, it is possible to allow, even in the absence of feeder cells or a coating material, pluripotent stem cells to proliferate with undifferentiation property thereof maintained and to form spheroids; and since pluripotent stem cells are allowed to proliferate in a closed system in which the risk of contamination is low, the safety can also be enhanced.

Patent Literature 8 discloses a method of maintaining and amplifying pluripotent stem cells, including repeating the steps of: (i) suspension culturing pluripotent stem cells until cell aggregates have an average diameter of about 200 - about 300 µm; and (ii) fragmenting the cell aggregates obtained by step (i) into uniform cell aggregates having an average diameter of about 80 - about 120 µm. In this method, the culture is performed in a medium containing a water-soluble polymer component having a viscosity that does not cause adhesion of cell aggregates, with use of a culture container such as a dish and in a stand-still state. However, in this method, the specific gravity of a medium is increased by use of a special medium, and the culture is performed in a state where cell aggregates float in the medium in a stand-still state. Therefore, this method has a problem that some molecules secreted by the cell aggregates will remain around the cell aggregates without flowing away, which influences the culture. In actuality, it has been pointed out that, when the diameter of cell aggregates exceeds 300 µm, a micro environment is formed due to an influence of cytokines and the like secreted by the cells, which induces differentiation, and in addition, necrosis occurs in the central part of the cell aggregates, which results in reduced recovery rate of variable cells. Furthermore, when the cell aggregates are subjected to filtration, it is necessary to cause the entire amount of the cell aggregate suspension medium to pass through a filter by use of a Pipetman. This requires a lot of manual work and is troublesome. In addition, it is difficult to separate the medium and the cell aggregates from each other, and replacing the medium requires much work.

### CITATION LIST

### [PATENT LITERATURE]

[PTL 1] International Publication WO2005/056072
[PTL 2] Japanese Unexamined Patent Application Publication No. 2009-159887
[PTL 3] International Publication WO2006/135103
[PTL 4] Japanese Unexamined Patent Application Publication No. 2008-237203
[PTL 5] Japanese Unexamined Patent Application Publication No. 2009-77708
[PTL 6] International Publication WO2010/143651
[PTL 7] International Publication WO2016/052657
[PTL 8] International Publication WO2013/077423

### [NON PATENT LITERATURE]

[NPL 1] Takahashi, K. and Yamanaka, S., (2006) Cell 126, p.663-676
[NPL 2] Takahashi K., et al., (2007) Cell 131, p.862-872
[NPL 3] Nakagawa M., et al. (2008) Nat. Biotechnol., 26(1): p.101-106
[NPL 4] Okita K., et al., (2011) Nat. Meth., p.409-412

### SUMMARY OF THE INVENTION

### PROBLEMS TO BE SOLVED BY THE INVENTION

In the cell culture method described in Patent Literature 7, spheroids of pluripotent stem cells formed through suspension culture are separated into small spheroids to be dispersed, and then the small spheroids are seeded in a culture medium and subcultured in a pseudo-microgravity environment such as rotary culture, whereby large-scale culture of pluripotent stem cells with the undifferentiated state thereof maintained is enabled. Here, it is disclosed that spheroids are mechanically (dynamically) disrupted into small spheroids (30 to 200 cells), typically through a filter. Specifically, spheroids are caused to pass through a filter by applying pressure with use of a pipette or the like.

In order to maintain the undifferentiated state of pluripotent stem cells, the culture is performed at a low cell density of about 1 × 10⁴ to 1 × 10⁵ cells/cm³. However, in order to perform large-scale culture to attain the number of cells in the order of 10⁹ to 10¹⁰, if a vessel having a volume of about 50 ml is used and proliferation by 3- to 5-fold is attained in one culture, it is necessary to perform subculture 5 to 6 times, and each subculture requires operations such as recovery of cultured cells (condensation), filtering process, dispensing of cells and addition of culture medium (dilution), and injection into a culture vessel. The method of manually causing the spheroids to pass through a filter to fine the spheroids, and diluting the resultant spheroids with a culture medium to perform subculture not only requires a lot of work but also causes large variation due to the skills of operators, and cannot eliminate the risk of contamination.

In consideration of the above-described circumstances, an object of the present invention is to provide: a large-scale cell culture system which can perform, even in the absence of feeder cells or a coating material, large-scale culture of pluripotent stem cells, in particular, iPS cells, to be used in regenerative medicine or the like, while maintaining undifferentiation property thereof, which can perform subculture while eliminating variation due to skills of operators, and which is also suitable for performing large-scale culture of adhesive cells in a suspended state without allowing adhesion thereamong; an inter-vessel cell liquid transfer device to be used therein; and a rotary cell culture device.

### SOLUTION TO THE PROBLEMS

In order to solve the above-described problems, the present invention provides a large-scale cell culture system, an inter-vessel cell liquid transfer device to be used therein, and a rotary cell culture device described below.
(1) A large-scale cell culture system for performing large-scale culture of pluripotent stem cells or adhesive cells by performing, in a closed system, subculture and transfer of spheroids and a culture medium by use of a vessel having a syringe structure, wherein the vessel includes a front flange and a back flange which have a same circular outer shape and which are provided integrally with both ends of an outer cylinder part of the vessel, and the vessel allows rotary culture, utilizing the front flange and the back flange in a state where a head, of the vessel, serving as a port for a solution is closed by a detachable cap and a space, in the vessel, closed by a gasket of a plunger is filled with a cell liquid obtained by suspending cells in a culture medium.
(2) The large-scale cell culture system according to (1), wherein a shaft part of the plunger is able to be separated at a halfway point thereof.
(3) The large-scale cell culture system according to (1) or (2), wherein an inner face at a distal end side of the outer cylinder part is formed as a conically recessed portion, and an inclination angle α of the inner face is set in a range of 80° to 160° in terms of a central angle.
(4) The large-scale cell culture system according to any one of (1) to (3), wherein a process is performed between subcultures, the process including a cell condensation step of transferring spheroids obtained through culture, from a culture vessel to a condensation vessel, and in the cell condensation step, a head of the culture vessel and a head of the condensation vessel are coupled to each other through a connection tool, the coupled culture vessel and condensation vessel are attached to an inter-vessel cell liquid transfer device in a state where the head of the culture vessel is oriented downward, the inter-vessel cell liquid transfer device being capable of holding outer cylinder parts of the respective vessels and capable of performing driving so as to push and pull a plunger, and an entire amount of settled spheroids is transferred from the culture vessel to the condensation vessel.
(5) The large-scale cell culture system according to (4), wherein a volume of the condensation vessel is set to be smaller than a volume of the culture vessel.
(6) The large-scale cell culture system according to (4) or (5), wherein a process is performed between subcultures, the process including a dispensing-fining step of transferring a predetermined amount of the cell liquid, in which the spheroids have been condensed, in the condensation vessel into a plurality of new culture vessels, wherein in a state where the head of the condensation vessel holding the cell liquid in a condensed state and a head of a new culture vessel are connected to each other through the connection tool having a filter built therein, the condensation vessel and the new culture vessel are attached to the inter-vessel cell liquid transfer device, simultaneously with a plunger of the condensation vessel being pushed in, a plunger of the culture vessel is pulled, and a predetermined amount of the cell liquid, in which the spheroids have been condensed, in the condensation vessel is caused to pass through the filter to be transferred into the culture vessel, the filter having a function of disrupting spheroids into small spheroids.
(7) The large-scale cell culture system according to (6), wherein a process is performed between subcultures, the process including a dilution step of adding a new culture medium into the culture vessel into which the predetermined amount of the cell liquid, in which the spheroids have been condensed, has been transferred, wherein a culture medium is supplied into the culture vessel from a supply source of a new culture medium, the supply source being connected to the head of the culture vessel.
(8) An inter-vessel cell liquid transfer device capable of transferring a solution from one vessel to another vessel, wherein the vessel includes a front flange and a back flange which have a same circular outer shape and which are provided integrally with both ends of an outer cylinder part of the vessel, the vessel allows rotary culture, utilizing the front flange and the back flange in a state where a head, of the vessel, serving as a port for a solution is closed by a detachable cap and a space, in the vessel, closed by a gasket of a plunger is filled with a cell liquid obtained by suspending cells in a culture medium, the inter-vessel cell liquid transfer device includes: a fixation part provided with an outer cylinder part holder which fixes outer cylinder parts of two respective vessels in a state where heads of the vessels are connected to each other through a connection tool; a movable part which moves a plunger holder which holds a plunger button provided at a distal end of a plunger; and a drive mechanism part which drives the movable part.
(9) A rotary cell culture device capable of rotating, at a predetermined speed, a culture vessel filled with a cell liquid obtained by suspending cells in a culture medium, the rotary cell culture device capable of performing culture of cells in a suspended state in a pseudo-microgravity environment, wherein the vessel includes a front flange and a back flange which have a same circular outer shape and which are provided integrally with both ends of an outer cylinder part of the vessel, in a state where a head, of the vessel, serving as a port of a solution is closed by a detachable cap and a space, in the vessel, closed by a gasket of a plunger is filled with the cell liquid, each of lower portions of the front flange and the back flange is rotatably supported by a pair of rollers, and at least one of the rollers that are in contact with the front flange or the back flange is a driving roller which is rotated by a drive motor, and an other of the rollers that are in contact with the front flange or the back flange is a driven roller, and the rotary cell culture device includes a restriction part for keeping an attitude by contacting and stopping an outer face extending in a radial direction of the front flange and the back flange.
(10) The rotary cell culture device according to (9), wherein in order to enable simultaneous rotary culture of a plurality of the culture vessels, a plurality of sets of the driving roller, the driven roller, and the restriction part are provided on a base.

### ADVANTAGEOUS EFFECTS OF THE INVENTION

According to the present invention, an efficient and large-scale culture of pluripotent stem cells or adhesive cells to be used in regenerative medicine or the like can, without being influenced by the skills of operators, be performed. Since the culture vessel has a syringe structure, all the condensation, filtering process, dilution, and rotary culture of cells in subculture can be performed in a closed system, and the risk of contamination is low. In the filtering process, cultured cells are simply transferred into another vessel through a connection tool having a filter built therein, whereby spheroids can be mechanically fined into small spheroids without using any agent liquid. In particular, a large-scale culture of iPS cells can be performed with the undifferentiation property thereof maintained.

### BRIEF DESCRIPTION OF THE DRAWINGS

[FIG. 1] FIG. 1 shows vessels used in a large-scale cell culture system of the present invention, wherein FIG. 1A is a partial cross-sectional view of a culture vessel having a volume of 50 ml, and FIG. 1B is a partial cross-sectional view of a condensation vessel having a volume of 10 ml.
[FIG. 2] FIG. 2 is a cross-sectional view of a connection tool having a filter built therein, in an exploded state.
[FIG. 3] FIG. 3 is a cross-sectional view of the connection tool having a filter built therein, in an assembled state.
[FIG. 4] FIG. 4 is a partial enlarged cross-sectional view showing a state in which the head of a culture vessel and the head of a condensation vessel are connected to each other though a connection tool having a filter built therein.
[FIG. 5] FIG. 5 shows a cell condensation step of transferring spheroids obtained through culture, from a culture vessel to a condensation vessel, wherein FIG. 5A is a cross-sectional view showing a state in which the head of the culture vessel and the head of the condensation vessel are connected to each other through a connection tool, and FIG. 5B is a cross-sectional view showing a state in which spheroids having settled in a lower part of the culture vessel have been transferred to the condensation vessel.

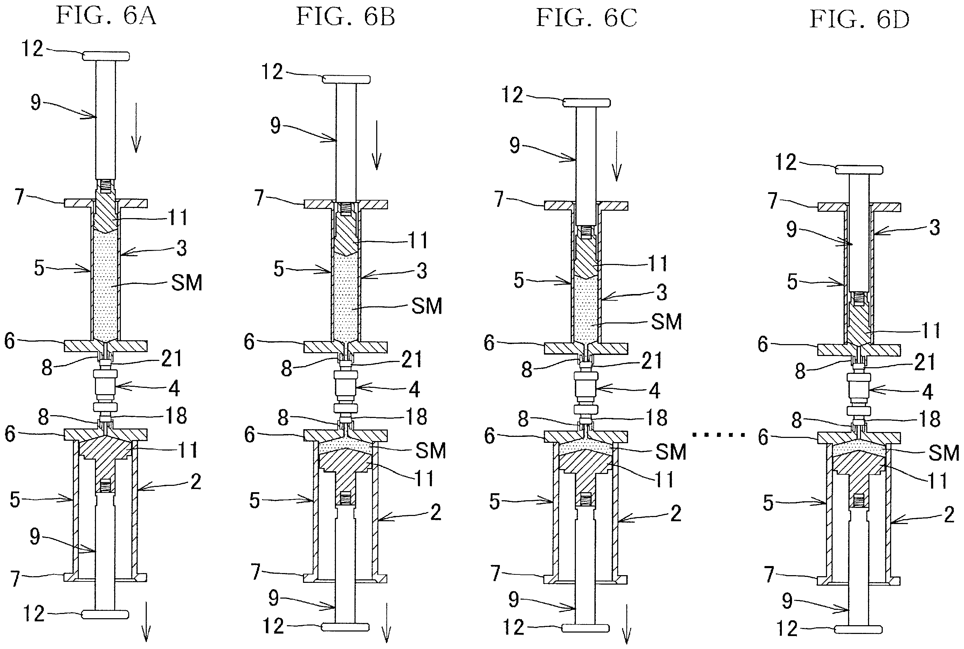

[FIG. 6] FIG. 6 shows a dispensing-fining step of dispensing spheroids while fining the spheroids into small spheroids, with the head of the condensation vessel and the head of a new culture vessel being connected to each other through a connection tool having a filter built therein, wherein FIG. 6A is a cross-sectional view showing a state in which the head of the condensation vessel and the head of a first culture vessel are connected to each other, FIG. 6B is a cross-sectional view showing a state in which 1/n of the spheroids have been transferred into the first culture vessel, FIG. 6C is a cross-sectional view showing a state in which 1/n of the spheroids have been transferred into a second culture vessel, and FIG. 6D is a cross-sectional view showing a state in which 1/n of the spheroids have been transferred into an n-th culture vessel.
[FIG. 7] FIG. 7 shows a dilution step of adding a new culture medium into the culture vessel into which 1/n of the spheroids have been transferred, wherein FIG. 7A is a cross-sectional view showing a state in which a culture medium supply tube is connected to the head of the culture vessel, FIG. 7B is a cross-sectional view showing a state in which the culture medium has been injected into the culture vessel, and FIG. 7C is a cross-sectional view showing a mode of a rotary culture vessel in which a cap is attached to the head thereof and a shaft part of a plunger is separated.
[FIG. 8] FIG. 8 is a perspective view of the entirety of an inter-vessel cell liquid transfer device.
[FIG. 9] FIG. 9 is a perspective view showing a state in which vessels are to be set in the inter-vessel cell liquid transfer device.
[FIG. 10] FIG. 10 is a partial longitudinal cross-sectional view of the inter-vessel cell liquid transfer device.
[FIG. 11] FIG. 11 is a partial longitudinal side view of the inter-vessel cell liquid transfer device in a case where a cell liquid is transferred from a culture vessel to a condensation vessel.
[FIG. 12] FIG. 12 is a partial longitudinal side view of the inter-vessel cell liquid transfer device in a case where a cell liquid is transferred from a condensation vessel to a culture vessel.
[FIG. 13] FIG. 13 is a perspective view of a rotary cell culture device.
[FIG. 14] FIG. 14 is a partial perspective view showing a drive mechanism of the rotary cell culture device.
[FIG. 15] FIG. 15 is a cross-sectional view showing another embodiment of the syringe (culture vessel).
[FIG. 16] FIG. 16 is a cross-sectional view showing another embodiment of the connection tool.
[FIG. 17] FIG. 17 is a perspective view showing a rotary cell culture device of a second embodiment.

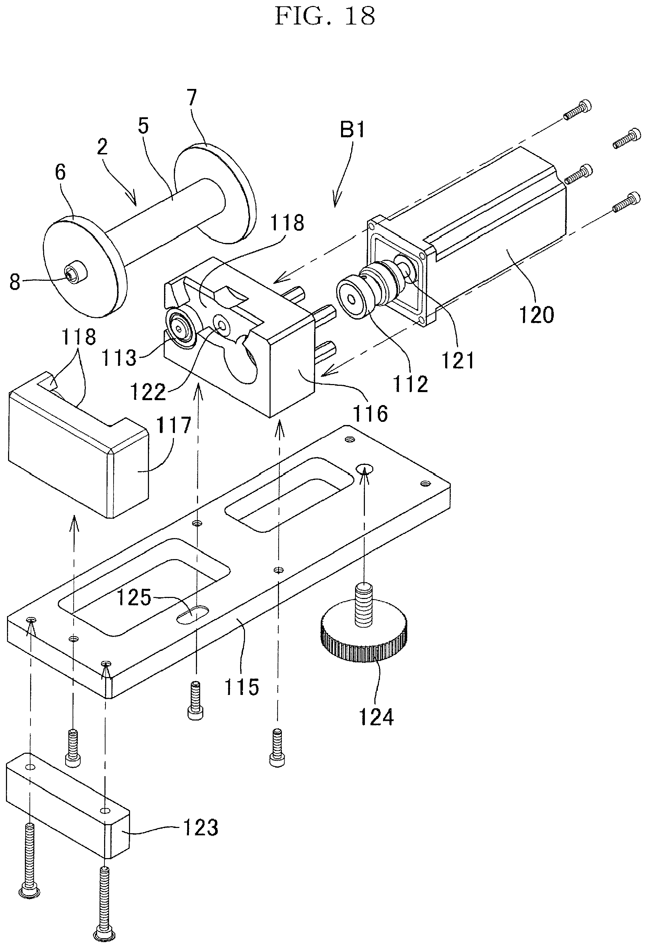

[FIG. 18] FIG. 18 is an exploded perspective view of the rotary cell culture device of the second embodiment.

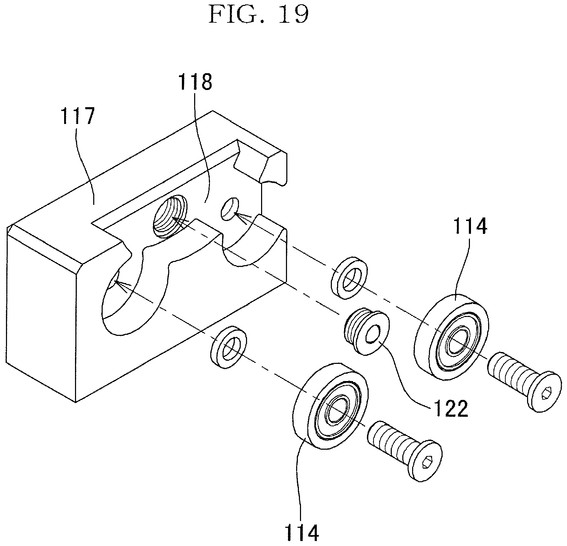

[FIG. 19] FIG. 19 is a partial exploded perspective view of the rotary cell culture device of the second embodiment.

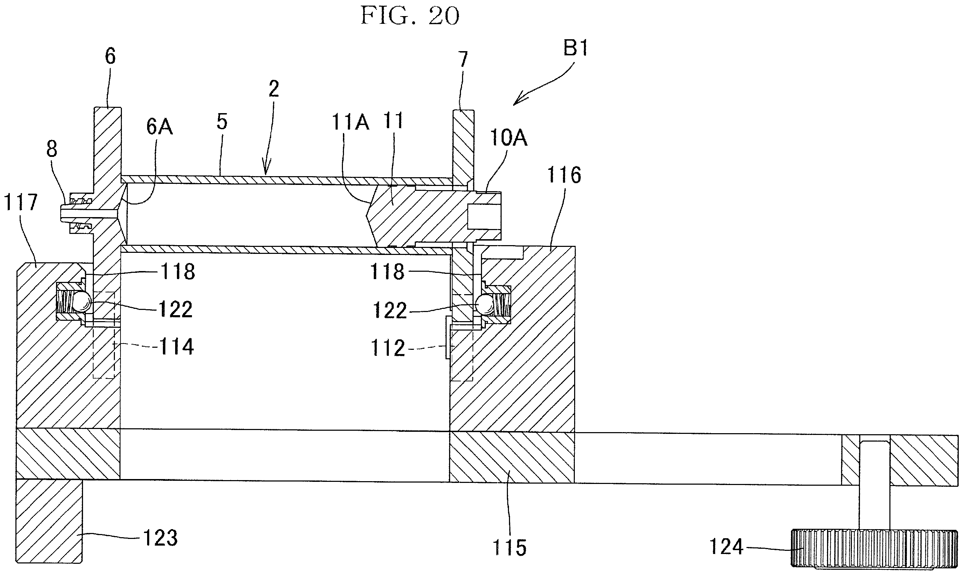

[FIG. 20] FIG. 20 is a cross-sectional view of FIG. 17 taken along the line X-X.
[FIG. 21] FIG. 21 is an exploded perspective view of a rotary cell culture device according to a modification of the second embodiment.
[FIG. 22] FIG. 22 is a perspective view showing a rotary cell culture device of a third embodiment in which three rotary cell culture devices of the second embodiment are mounted.
[FIG. 23] FIG. 23 is a partial exploded perspective view of the rotary cell culture device of the third embodiment.
[FIG. 24] FIG. 24 is a perspective view showing a rotary cell culture device of a fourth embodiment.
[FIG. 25] FIG. 25 is a cross-sectional view of FIG. 24 taken along the line Y-Y.
[FIG. 26] FIG. 26 is a cross-sectional view of FIG. 24 taken along the line Z-Z.
[FIG. 27] FIG. 27 is a partial perspective view showing a drive mechanism.
[FIG. 28] FIG. 28 shows phase contrast images of small spheroids having passed through a 70 µm filter, wherein FIG. 28A is a low magnification phase contrast image, and FIG. 28B is a high magnification phase contrast image.
[FIG. 29] FIG. 29 shows phase contrast images of spheroids obtained by culturing small spheroids in FIG. 15, wherein FIG. 29A is a low magnification phase contrast image, and FIG. 29B is a high magnification phase contrast image.
[FIG. 30] FIG. 30 shows phase contrast images of spheroids obtained through 3 days rotary culture of iPS cells (253G1 cells) using 10 ml vessels, wherein FIG. 30A is a phase contrast image of spheroids obtained through rotary culture in an mTeSR1 medium, and FIG. 30B is a phase contrast image of spheroids obtained through rotary culture in an AK02N medium.
[FIG. 31] FIG. 31 shows phase contrast images of spheroids obtained through 3 days rotary culture of iPS cells (409B2 cells) using 10 ml vessels, wherein FIG. 31A is a phase contrast image of spheroids obtained through rotary culture in an mTeSR1 medium, and FIG. 31B is a phase contrast image of spheroids obtained through rotary culture in an AK02N medium.
[FIG. 32] FIG. 32 is a phase contrast image of small spheroids observed immediately after disruption at each passage (PI to PI0) in a serially passaged culture experiment.

### DESCRIPTION OF EMBODIMENTS

In the present invention, a "cell" means a "cultured cell", and is a pluripotent stem cell such as an ES cell or an iPS cell, or an adhesive cell. Cells used in the present invention may be derived from any mammal (for example, mouse, rat, guinea pig, hamster, rabbit, dog, cat, monkey, cattle), but more preferably, are iPS cells derived from human.

The medium (culture medium) in which cells are suspended is not limited in particular as long as the medium is suitable for culturing the cells. FBS (fetal bovine serum) or an antibiotic such as Antibiotic-Antimycotic may be added to the medium. The culture medium used in the large-scale cell culture system in the present invention is such a culture medium in which when the culture medium is left to stand still, spheroids mixed in a dispersed state sediment and accumulate in a lower portion.

In the present invention, a "spheroid" means a cell aggregate formed by a large number of cells being aggregated, and means a cell aggregate typically having a diameter of not less than 300 µm, for example, 300 to 2000 µm. In the present invention, a "small spheroid" means a cell aggregate having a relatively small size and composed of a relatively small number of cells, and includes a cell aggregate typically composed of about 3 to 1,000 cells, for example, a cell aggregate composed of 5 to 600 cells, 10 to 200 cells, 30 to 100 cells, or 20 to 40 cells.

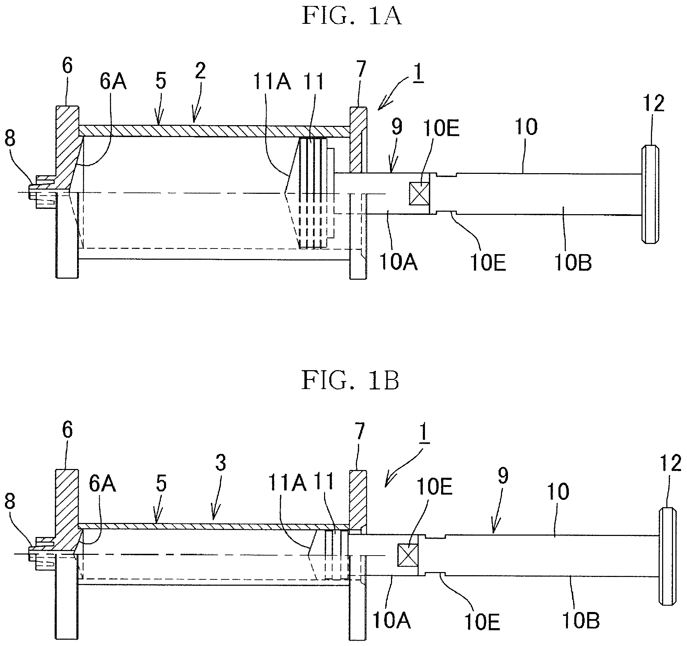

Next, with reference to the embodiments shown the attached drawings, the present invention is described further in detail. FIG. 1 to FIG. 7 show vessels used in culture according to the present invention, and the procedure of large-scale cell culture using the vessels. In the drawings, the reference character 1 denotes a syringe, the reference character 2 denotes a culture vessel, the reference character 3 denotes a condensation vessel, and the reference character 4 denotes a connection tool. The reference character S denotes cells or spheroids, the reference character M denotes a culture medium, and the reference character SM denotes a cell liquid obtained by suspending cells or spheroids in a culture medium.

As shown in FIG. 1, the syringe 1 is structured such that: a front flange 6 and a back flange 7 which have the same circular outer shape are provided integrally with both ends of an outer cylinder part 5 having a cylindrical shape; a head 8 provided at a center portion of the front flange 6 and serving as a discharge port has a standard Luer lock structure; a gasket 11 slidably and elastically fitted in the outer cylinder part 5 is provided at the distal end of a shaft part 10 of a plunger 9; and a plunger button 12 is provided at the proximal end.

Here, the shaft part 10 of the plunger 9 can be separated into a short shaft part 10A at the distal end side and a long shaft part 10B at the proximal end side. The present embodiment employs a structure in which a female screw 10C is provided at an end portion of the shaft part 10A, and a male screw 10D to be screwed into the female screw 10C is formed at an end portion of the shaft part 10B, so as to allow engagement and disengagement between the shaft part 10A and the shaft part 10B. Further, in order to allow relative rotation between the shaft part 10A and the shaft part 10B at the time of engagement and disengagement thereof, a notch 10E is formed at the outer face of each of the shaft part 10A and the shaft part 10B. A distal end tip 11A of the gasket 11 has a conically protruding shape, and the inner face at the distal end side of the outer cylinder part 5, i.e., an inner face 6A of the front flange 6, has a conically recessed shape so as to be able to receive the distal end tip 11A and discharge all the liquid in the outer cylinder part 5. The head 8 made of stainless steel and having a Luer lock structure is formed integrally with the outer face of the front flange 6.

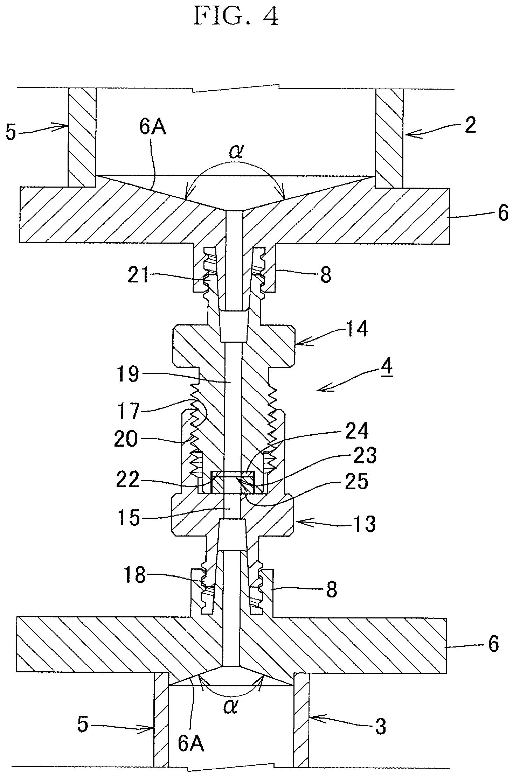

Here, the inclination angle of the inner face 6A having a tapered shape is important so as to cause spheroids that have sedimented as described later, to be efficiently discharged from the head 8 together with a small amount of culture medium. In the present invention, as shown in FIG. 4, an inclination angle α of the inner face 6A is set, in terms of a central angle, in a range of 80° to 160°, more preferably in a range of 90° to 150°. If the inclination angle of the inner face 6A is too large, when the spheroids are caused to sediment with the shaft vertically oriented, the spheroids are less likely to gather in the vicinity of the discharge port of the head 8. Meanwhile, if the inclination angle of the inner face 6A is too small, when rotary culture is performed with the shaft horizontally oriented, since the angular velocity of the culture medium induced by the inner face 6A differs depending on the radius, uneven distribution of the spheroids could be caused, and observation of the part near the inner face 6A is difficult. In the present embodiment, the distal end tip 11A has a conically protruding shape, but may have the same inclination angle as or a greater inclination angle than the inner face 6A, or may be flat (180°). In the syringe 1 shown in FIG. 1 and FIG. 4, the inclination angle α of the inner face 6A is 150° in terms of a central angle.

The syringe 1 is made from a material which can be subjected to a bactericidal process such as autoclave sterilization. In the present embodiment, the outer cylinder part 5 is made from transparent Pyrex glass so as to allow observation of the suspended state or sedimented state of the spheroids therein, the front flange 6, the back flange 7, and the shaft part 10 of the plunger 9 are made from stainless steel, and the gasket 11 of the plunger 9 is made from PTFE. However, the materials of the syringe 1 are not limited to those mentioned above, and, similar to the outer cylinder part 5, the front flange 6 and the back flange 7 may be made from Pyrex glass, or may be molded from a transparent synthetic resin material. In a case where the outer cylinder part 5, the front flange 6, and the back flange 7 of the syringe 1 are molded from synthetic resin, the entirety thereof may be integrally molded, or may be molded as a plurality of divided parts and the obtained parts may be joined together through adhesion, heat welding, or ultrasonic welding.

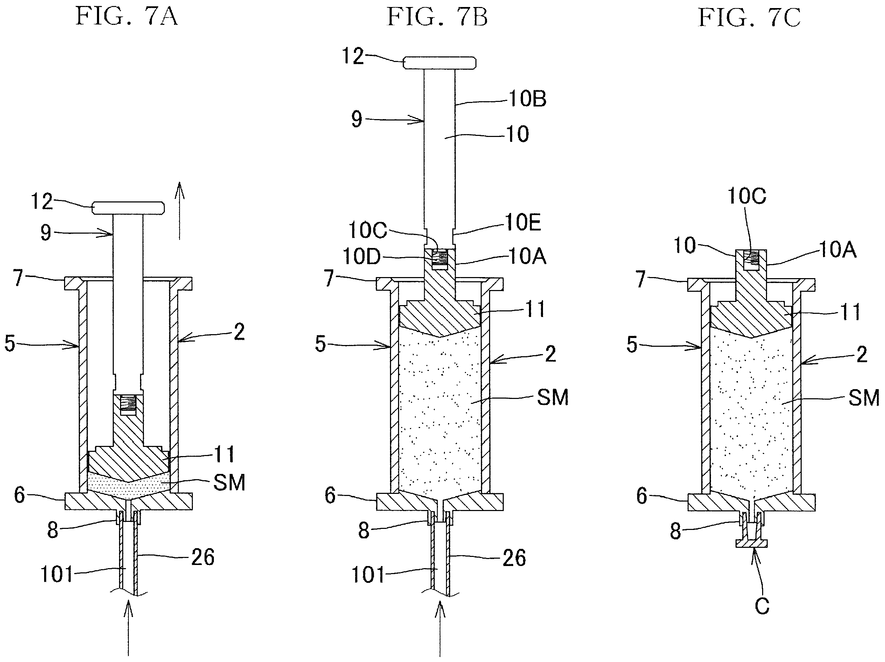

As the culture vessel 2, the syringe 1 is used as is. However, when the syringe 1 is attached to a rotary cell culture device described later to perform rotary culture, the syringe 1 is used in a state where a cap C is attached to the head 8 and the shaft part 10B of the plunger 9 is separated, as shown in FIG. 7C. In the present invention, the syringe 1 and the culture vessel 2 are synonymous and are used without distinction. However, in a narrow sense of the culture vessel 2 that is used in rotary culture, the culture vessel 2 takes the form shown in FIG. 7C. The culture vessel 2 shown in FIG. 1A has a volume of 50 ml, the diameter of each of the front flange 6 and the back flange 7 is 50 mmφ, and the dimension between the outer shapes of the front flange 6 and the back flange 7 is 91 mm. The stroke of the plunger 9 is about 60 mm. The culture vessel 2 is used in rotary culture, in a state where the head 8 is closed by the cap C and the space closed by the gasket 11 of the plunger 9 is filled with a cell liquid obtained by suspending cells in a culture medium.

The syringe 1 shown in FIG. 1B has the same structure and outer shape dimensions as the syringe 1 shown in FIG. 1A, except that the syringe 1 shown in FIG. 1B has an outer cylinder part 5 and a gasket 11 of which diameters are smaller than those in the syringe 1 shown in FIG. 1A and has a volume of 10 ml. This syringe 1 is used as a condensation vessel 3 in the present invention, and is used in order to transfer and dispense spheroids obtained through culture, from the culture vessel 2 into a plurality of new other culture vessels 2. However, as a matter of fact, this syringe 1 can be used as a culture vessel having a small volume. Since the structure of the condensation vessel 3 is the same as that of the culture vessel 2, the same components are denoted by the same reference characters, respectively, and description thereof is omitted.

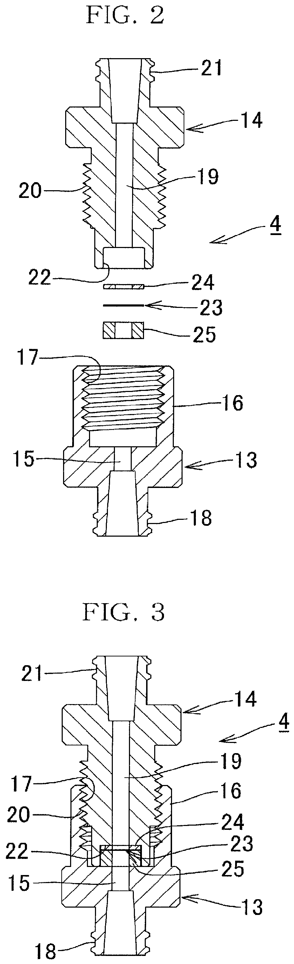

As shown in FIG. 2 to FIG. 4, the connection tool 4 is used when the culture medium is transferred between the culture vessel 2 and the condensation vessel 3, and can connect the heads 8, 8 of the respective vessels 2 and 3 to each other. The connection tool 4 is composed of a first member 13 and a second member 14. In the first member 13, a flow path 15 is provided at the center thereof, a hollow housing part 16 is provided at one end side in the axial direction, a female screw part 17 is provided at the inner periphery of the housing part 16, and a connection part 18 to be connected to the head 8 is provided at the other end side in the axial direction. In the second member 14, a flow path 19 is provided at the center thereof, a male screw part 20 to be screwed into the female screw part 17 of the first member 13 is provided at one end side in the axial direction, and a connection part 21 to be connected to the head 8 is provided at the other end side in the axial direction. A recessed portion 22 is formed at the distal end of the male screw part 20 of the second member 14. A filter 23, a packing 24, and a spacer 25 are disposed in a stacked manner in the recessed portion 22, and are compressed and sealed between the inner face extending in the radial direction of the housing part 16 of the first member 13 and the inner face extending in the radial direction of the recessed portion 22 of the second member 14, in a state where the male screw part 20 of the second member 14 is screwed into the female screw part 17 of the first member 13. The packing 24 and the spacer 25 each has a center hole that allows communication with the flow paths 15 and 19. Although not shown, the filter 23 may be interposed between two packings 24, 24.

As the filter 23, a filter having a filtration particle size that allows disruption of the generated spheroids into small spheroids having smaller sizes can be used, and filters having a filtration particle size of 40 to 100 µm, and preferably 60 to 80 µm, for example, 70 µm are preferable. The small spheroids obtained as a result of disruption through the filter have slender shapes in general. Spheroids may be disrupted through the filter 23 once or twice or more. When the disruption through the filter is performed twice or more, filters 23 having the same filtration particle size may be used or filters 23 having different filtration particle sizes may be used, at the filtration. For example, after spheroids having been disrupted through the filter 23, the disrupted spheroids (small spheroids) may be further disrupted through a filter 23 having a finer filtration particle size, whereby the sizes (major axis, in particular) of the obtained small spheroids can be caused to fall in a smaller range. In the present embodiment, a 200 mesh stainless steel filter is used as the filter 23, and the filtration particle size thereof is 70 µm.

Through the use of the connection tool 4 having the filter 23 built therein, the cell liquid which contains spheroids is transferred from a vessel to another vessel, and at the same time, the spheroids are disrupted into small spheroids having sizes according to the filtration particle size as a result of the spheroids passing through the filter 23. In a case where the cell liquid is simply transferred from a vessel to another vessel without the spheroids being disrupted, a connection tool 4 without the filter 23 may be used.

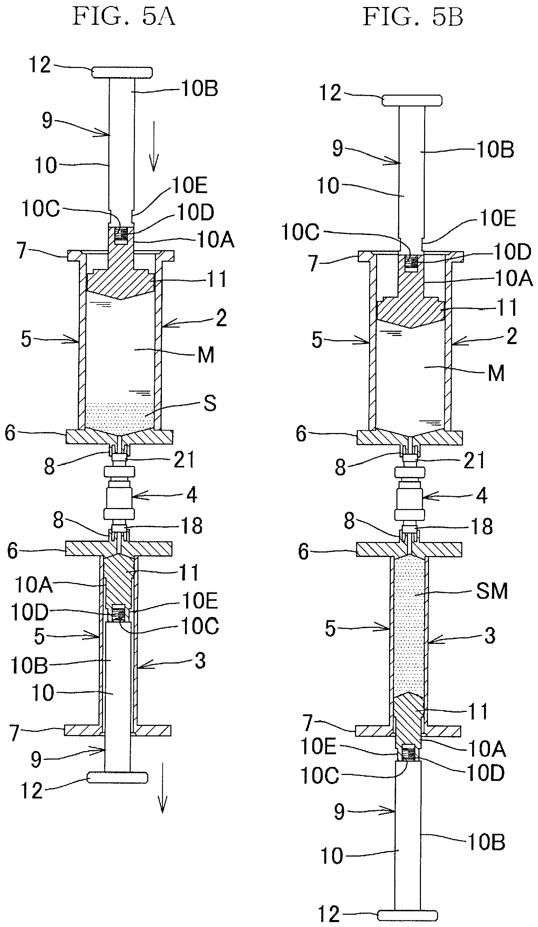

Next, the outline of the large-scale cell culture system of the present invention is described with reference to FIG. 5 to FIG. 7. First, a process is described that is performed after: a predetermined number of cells and a culture medium are injected into the culture vessel 2; a cell liquid of about 1 × 10⁴ to 1 × 10⁵ cells/cm³ is prepared; and the number of cells has increased about 5 times through proliferation in a 3-day culture performed in a rotary cell culture device B described later. As the cultured cells, iPS cells (253G1) are selected, and an mTeSRI (containing Y27632) medium is used. From ALP staining, mRNA expression of an undifferentiation marker, and a result of flow cytometry of the obtained spheroids, it has been confirmed that the cultured cells retain the undifferentiation property as that of iPS cells and have differentiation potency into three germ layers.

First, with reference to FIG. 5, a cell condensation step in which spheroids obtained through culture are transferred from a culture vessel to a condensation vessel is described. As shown in FIG. 5A, the shaft part 10A of the plunger 9 of the culture vessel 2 is connected to the shaft part 10B having been separated during the culture, to form a syringe having a normal structure, the cap C is removed from the head 8 of the culture vessel 2, this head 8 is connected to one connection part 21, without a filter, of the connection tool 4, the head 8 of the condensation vessel 3 is connected to the other connection part 18 of the connection tool 4, and the resultant syringe is disposed with the head 8 of the culture vessel 2 oriented downward and is left to stand still. Since the connection tool 4 is structured so as to allow two-way usage, the connection part 18 may be connected to the head 8 of the culture vessel 2, and the connection part 21 may be connected to the head 8 of the condensation vessel 3.

When the syringe is left to stand still in the state shown in FIG. 5A, spheroids S settle in a lower part at the head 8 side of the culture vessel 2. In this state, as shown in FIG. 5B, the plunger 9 of the culture vessel 2 is pushed in and the plunger 9 of the condensation vessel 3 is pulled, such that the entire amount of the spheroids S in the culture vessel 2 is transferred to the condensation vessel 3 together with a culture medium M. Inside the condensation vessel 3, the volume is reduced to 1/5 in a state where the number of cells has increased about 5 times through proliferation, whereby a cell liquid SM in a condensed state is obtained.

Next, with reference to FIG. 6, a dispensing-fining step is described in which the heads 8, 8 of the condensation vessel 3 and a new culture vessel 2 are connected to each other through the connection tool 4 having a filter built therein, and the spheroids are dispensed while being fined into small spheroids. As shown in FIG. 6A, the head 8 of the condensation vessel 3 holding the cell liquid SM in the condensed state and the head 8 of a first new culture vessel 2 are connected to each other by means of the connection tool 4 having a filter built therein. Then, as shown in FIG. 6B, the plunger 9 of the condensation vessel 3 is pushed in and the plunger 9 of the culture vessel 2 is pulled, such that 1/5 of the cell liquid SM, in which the spheroids S have been condensed, in the condensation vessel 3 is transferred to the culture vessel 2. Similarly, as shown in FIG. 6C, a second new culture vessel 2 is connected, and 1/5 of the cell liquid SM in the condensation vessel 3 is transferred to the culture vessel 2. Then, as shown in FIG. 6D, a fifth new culture vessel 2 is connected, and 1/5 of the cell liquid SM in the condensation vessel 3 is transferred to the culture vessel 2.

When the cell liquid SM is transferred through the filter 23 from the condensation vessel 3 to the culture vessel 2, the spheroids are disrupted into small spheroids by the filter 23. In the cell condensation step shown in FIG. 5, since the connection tool 4 having a filter built therein is used, the spheroids are disrupted into small spheroids by the filter 23 also in this step. That is, the filtering process can be performed twice, after the second generation culture and before the next n+1 th generation culture, and thus, the small spheroids can be made more spherical.

Next, with reference to FIG. 7, a dilution step is described in which a new culture medium M is added to the culture vessel 2 into which 1/5 of the cell liquid SM, in which the spheroids S have been condensed, has been transferred. As shown in FIG. 7A, a culture medium supply tube 26 is connected to the head 8 of the culture vessel 2 into which the cell liquid SM containing 1/5 of the condensed spheroids has been transferred in the dispensing-fining step, and then, as shown in FIG. 7B, the culture medium M is sucked while the plunger 9 is being pulled, whereby a new culture medium M is added to dilute the mixture. As another method for adding a new culture medium into the culture vessel 2, a configuration may be employed in which a syringe (not shown) holding a new culture medium is prepared, and the syringe is connected to the culture vessel 2 through the connection tool 4, and then the culture medium is injected from the syringe. In this case, all of the cell condensation step, the dispensing-fining step, and the dilution step described above can be performed in a closed system by use of an inter-vessel cell liquid transfer device A described later.

Finally, after the condensed spheroids are diluted with the culture medium, the cap C is attached to the head 8 of the culture vessel 2 as shown in FIG. 7C to close the culture vessel 2, and the shaft part 10B of the plunger 9 is separated from the shaft part 10A, whereby the culture vessel 2 is made ready to be attached to the rotary cell culture device B.

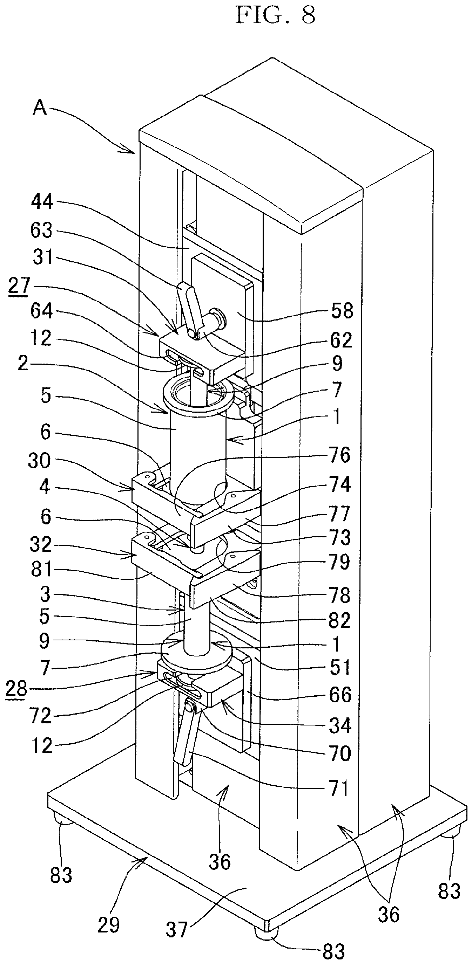

Next, with reference to FIG. 8 to FIG. 12, the inter-vessel cell liquid transfer device A of the present invention is described. The inter-vessel cell liquid transfer device A is an automated device, in which, in a state where the heads 8, 8 of two vessels are connected to each other by means of the connection tool 4, the outer cylinder part 5 is fixed, specifically, the back flange 7 is held so as not to be able to move in the axial direction, the plunger 9 is driven in the axial direction with the plunger button 12 gripped, and a predetermined amount of liquid can be transferred at a constant speed from one vessel to the other vessel.

The inter-vessel cell liquid transfer device A is provided with a first mechanism 27 and a second mechanism 28 which are opposed to each other in the same vertical direction and which are provided on a common base part 29, wherein vessels are attached to the first mechanism 27 and the second mechanism 28, respectively, and the plungers 9 are driven to advance and retreat in a state where the outer cylinder parts 5 are fixed. Here, in the present invention, "advance" means to travel in a direction in which liquid is discharged from the head 8 of a vessel, and "retreat" means to travel in a direction in which liquid is sucked from outside and to travel in an opposite direction to the advancement.

In the present embodiment, as shown in FIG. 8 to FIG. 11, an example is described in which the culture vessel 2 is attached to the first mechanism 27 and the condensation vessel 3 is attached to the second mechanism 28. Specifically, the first mechanism 27 is provided with: a fixation part 30 which fixes the outer cylinder part 5 of the culture vessel 2; a movable part 31 which drives the plunger 9 so as to advance and retreat; and a drive mechanism part 32 which drives the movable part 31. Meanwhile, the second mechanism 28 is also provided with: a fixation part 33 which fixes the outer cylinder part 5 of the condensation vessel 3; a movable part 34 which drives the plunger 9 so as to advance and retreat; and a drive mechanism part 35 which drives the movable part 34. Here, the movable part 31 and the drive mechanism part 32 of the first mechanism 27 and the movable part 34 and the drive mechanism part 35 of the second mechanism 28 have the same structures, and are arranged in an inversed manner in the up-down direction. The movable part 31 of the first mechanism 27 and the movable part 34 of the second mechanism 28 are driven so as to advance and retreat in a synchronized manner according to the cross sectional areas of the respective vessels such that the flow rates in the connection tool 4 match each other.

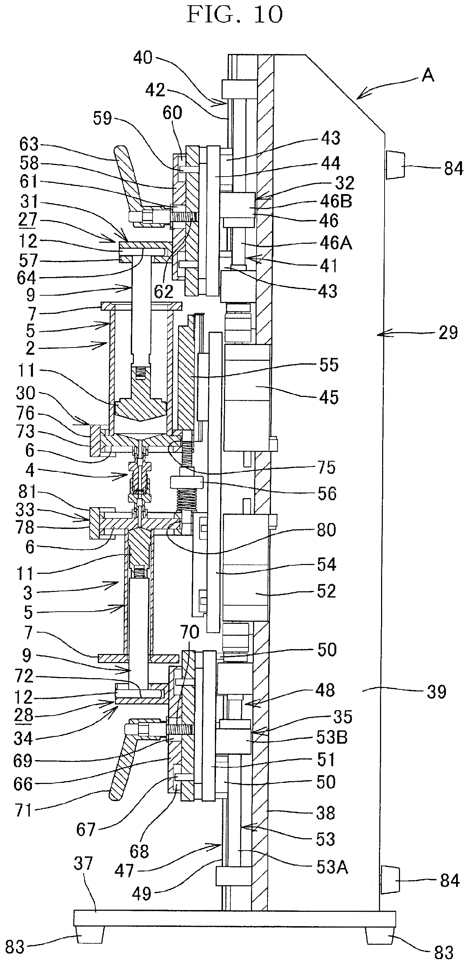

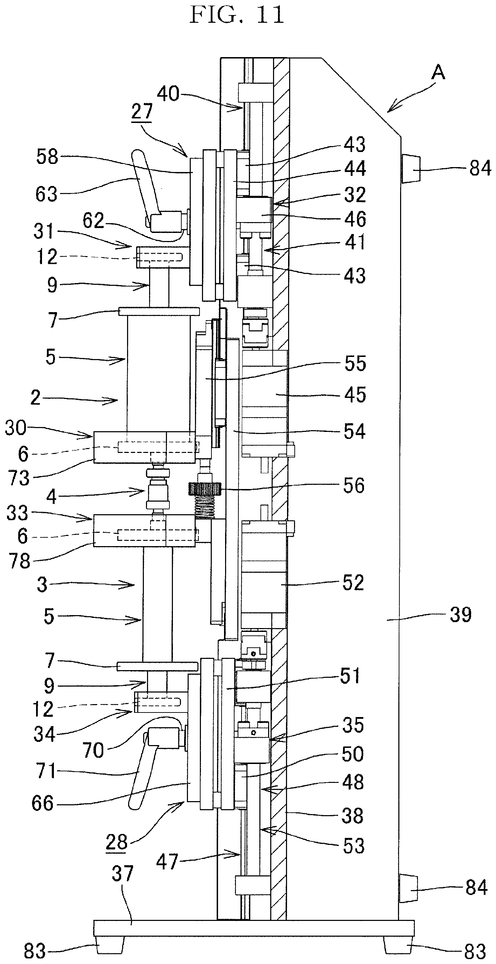

FIG. 10 and FIG. 11 each show the internal structure of the inter-vessel cell liquid transfer device A from which all the protection cover and the like 36 shown in FIG. 8 have been removed. As shown in FIG. 10, in the base part 29, a vertical support plate 38 is fixed, in a standing manner, to a center portion on the upper face of a horizontal base plate 37, and reinforcement plates 39, 39 are provided so as to stand along the both side edges at the rear face side of the support plate 38 and are fixed to the base plate 37 and the support plate 38, whereby a highly rigid structure having a U shape in a plan view is produced.

In an upper portion at the front face of the support plate 38, a linear guide 40 extending in the up-down direction and forming the drive mechanism part 32, and a linear actuator 41 provided along the linear guide 40 are disposed. The movable part 31 is held at the linear guide 40 so as to be movable in the up-down direction, and the movable part 31 is driven by the linear actuator 41. The linear guide 40 is composed of two parallel guide rails 42, 42, and the movable part 31 is fixed on a stage 44 provided with, on the rear face thereof, movable blocks 43, 43 which move along the guide rails 42, 42. The linear actuator 41 is implemented as a feed screw mechanism 46 which is driven to be rotated by a drive motor 45 which can control the rotation speed of a stepping motor, a servomotor, or the like. A screw shaft 46A of the feed screw mechanism 46 is disposed between and in parallel to the guide rails 42, 42, and a nut member 46B screwed to the screw shaft 46A so as to be able to advance and retreat with respect thereto is fixed to the rear face of the stage 44. Instead of the feed screw mechanism 46, a ball screw having a higher precision may be used.

Meanwhile, in a lower portion at the front face of the support plate 38, a linear guide 47 extending in the up-down direction and forming the drive mechanism part 35, and a linear actuator 48 provided along the linear guide 47 are disposed. The movable part 34 is held at the linear guide 47 so as to be moveable in the up-down direction, and the movable part 34 is driven by the linear actuator 48. The linear guide 47 is composed of two parallel guide rails 49, 49, and the movable part 34 is fixed on a stage 51 provided with, on the rear face thereof, movable blocks 50, 50 which move along guide rails 49, 49. The linear actuator 48 is implemented as a feed screw mechanism 53 which is driven to be rotated by a drive motor 52 which can control the rotation speed of a stepping motor, a servomotor, or the like. A screw shaft 53A of the feed screw mechanism 53 is disposed between and in parallel to the guide rails 49, 49, and a nut member 53B screwed to the screw shaft 53A so as to be able to advance and retreat with respect thereto is fixed to the rear face of the stage 51. Also in this case, instead of the feed screw mechanism 53, a ball screw having a higher precision may be used.

The fixation part 30 of the first mechanism 27 and the fixation part 33 of the second mechanism 28 are provided by being directly or indirectly mounted to a common fixation stage 54 which is fixed at the surface side of and in parallel to the support plate 38, and with an interval from the support plate 38. In the present embodiment, the fixation part 33 is directly mounted to a lower portion of the fixation stage 54; and the fixation part 30 is mounted to a movable stage 55 provided at an upper portion of the fixation stage 54 and movable in the up-down direction, and is coupled to the fixation part 30 through an interval adjuster 56 with respect to the fixation part 33. The interval adjuster 56 is a mechanism that expands and contracts due to a screw mechanism. The fixation part 30 is fixed to the fixation stage 54 through the interval adjuster 56 and the fixation part 33. The reason why the interval adjuster 56 is provided is to absorb errors in the dimensions of the connection tool 4 and in the connection depths of the connection tool 4 with respect to the heads 8 of the vessels.

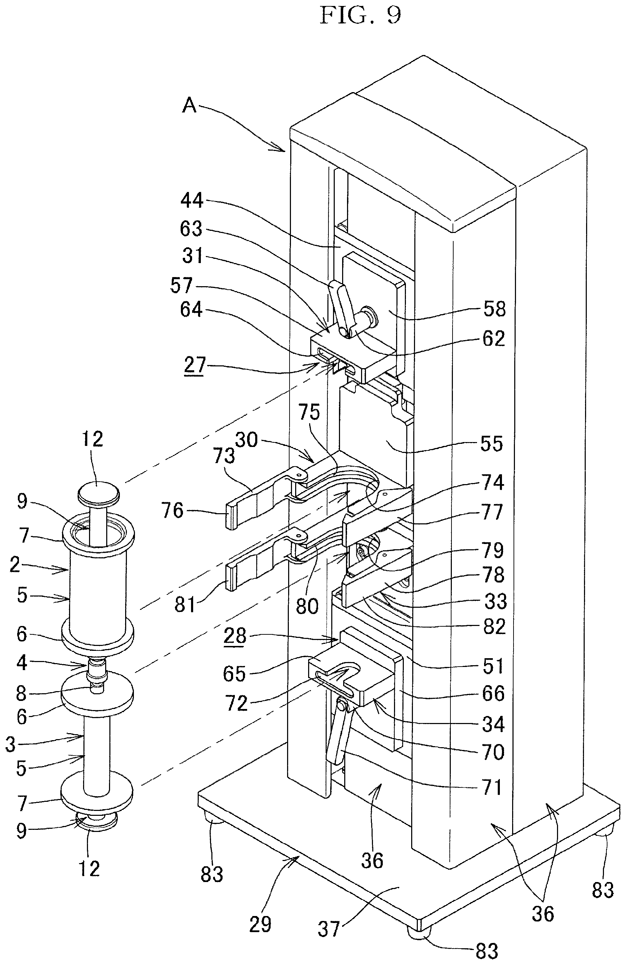

The movable part 31 of the first mechanism 27 is provided on the stage 44 and can fix a plunger holder 57 holding the plunger button 12 of the plunger 9 with respect to the stage 44, with the position of the plunger holder 57 slightly adjusted in the up-down direction. Specifically, in a state where an adjustment plate 58 is joined to a surface of the stage 44, the adjustment plate 58 is guided to move in the up-down direction by means of a pin 59 and a slit groove 60, and a fastening screw 62 is screwed into the stage 44 through a long hole 61 extending in the up-down direction and being open at the center of the adjustment plate 58. Fastening and loosening can be easily performed manually by using a handle 63 provided to the fastening screw 62. Here, as shown in FIG. 9, the plunger holder 57 is formed as an engagement recessed portion 64 which engages with the plunger button 12 from the front face side.

Similarly, the movable part 34 of the second mechanism 28 is provided on the stage 51 and can a fix a plunger holder 65 holding the plunger button 12 of the plunger 9 with respect to the stage 51, with the position of the plunger holder 65 slightly adjusted in the up-down direction. Specifically, in a state where an adjustment plate 66 is joined to a surface of the stage 51, the adjustment plate 66 is guided to move in the up-down direction by means of a pin 67 and a slit groove 68, and a fastening screw 70 is screwed into the stage 51 through a long hole 69 extending in the up-down direction and being open at the center of the adjustment plate 66. Fastening and loosening can be easily performed manually by using a handle 71 provided to the fastening screw 70. Here, as shown in FIG. 9, the plunger holder 65 is formed as an engagement recessed portion 72 which engages with the plunger button 12 from the front face side.

The fixation part 30 of the first mechanism 27 is provided with an outer cylinder part holder 73 which holds the outer cylinder part 5 of the culture vessel 2. Specifically, as shown in FIG. 9, the outer cylinder part holder 73 includes: a U-shaped recessed portion 74 which receives the outer cylinder part 5; a flange fitting groove 75 into which the front flange 6 fits; and a holding member 76 which presses and holds the front flange 6 from outside. The holding member 76 is configured such that one end thereof is held so as to be rotatable in the horizontal direction, and the other end thereof is flexibly engaged and disengaged by a hook 77. Here, it is also preferable that the outer cylinder part holder 73 can also hold the back flange 7 at the same time.

Similarly, the fixation part 33 of the second mechanism 28 is provided with an outer cylinder part holder 78 which holds the outer cylinder part 5 of the condensation vessel 3. Specifically, as shown in FIG. 9, the outer cylinder part holder 78 includes: a U-shaped recessed portion 79 which receives the outer cylinder part 5; a flange fitting groove 80 into which the front flange 6 fits; and a holding member 81 which presses and holds the front flange 6 from outside. The holding member 81 is configured such that one end thereof is held so as to be rotatable in the horizontal direction, and the other end thereof is flexibly engaged and disengaged by a hook 82. Here, it is also preferable that the outer cylinder part holder 78 can also hold the back flange 7 at the same time.

Although not shown, for safety and in order to determine the home position, a limiting mechanism which limits the movable range of each movable part is provided. The limiting mechanism is formed by a proximity sensor provided at a fixation portion, and a restriction piece provided at a movable portion. When the proximity sensor has detected the restriction piece, the drive motor 45, 52 is forcibly stopped. Further, ground pads 83 made of rubber are provided at the lower face of the base plate 37, and in addition, ground pads 84 made of rubber are provided at the edges of the reinforcement plates 39, 39, so that the inter-vessel cell liquid transfer device A can be also used in a state where the inter-vessel cell liquid transfer device A is laid sideways in the horizontal direction.

When the culture vessel 2 and the condensation vessel 3 are to be attached to the inter-vessel cell liquid transfer device A having the configuration described above, first, as shown in FIG. 9, in a state where the heads 8, 8 of the culture vessel 2 and the condensation vessel 3 are coupled to each other by means of the connection tool 4, the holding members 76, 81 are open, and the fastening screws 62, 70 are loosened, the front flange 6 of the culture vessel 2 is fitted into the flange fitting groove 75, the plunger button 12 is engaged with the engagement recessed portion 64, the front flange 6 of the condensation vessel 3 is fitted into the flange fitting groove 80, the plunger button 12 is engaged with the engagement recessed portion 72, and then the holding members 76, 81 are closed to hold the front flanges 6. Then, the fastening screws 62, 70 are fastened and set.

The states shown in FIG. 8 and FIG. 11 correspond to the state shown in FIG. 5A. This state is maintained for some time, thereby allowing spheroids to settle inside the culture vessel 2. Then, the drive motor 45 of the first mechanism 27 and the drive motor 52 of the second mechanism 28 are driven to rotate, whereby the movable part 31 is advanced to push in the plunger 9 of the culture vessel 2, and at the same time, the movable part 34 is retreated to pull the plunger 9 of the condensation vessel 3, and accordingly, a cell liquid, in which spheroids have been condensed, is transferred to the condensation vessel 3.

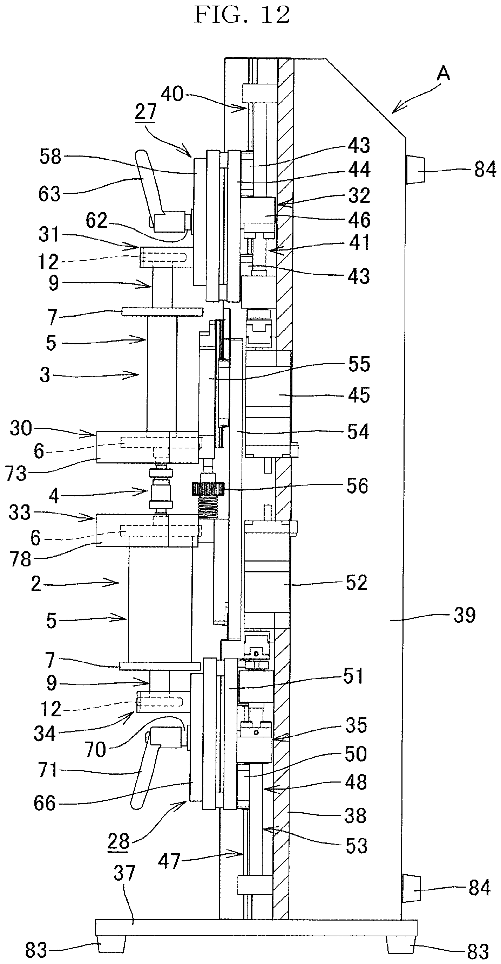

When the cell liquid, in which the spheroids have been condensed, in the condensation vessel 3 is to be dispensed into a new culture vessel 2, as shown in FIG. 12, in a state where a new culture vessel 2 is connected to the condensation vessel 3 through the connection tool 4 having a filter built therein, the new culture vessel 2 and the condensation vessel 3 are attached to the inter-vessel cell liquid transfer device A in a similar manner to that described above. The state shown in FIG. 12 corresponds to the state shown in FIG. 6A. Also in this case, the drive motor 45 of the first mechanism 27 and the drive motor 52 of the second mechanism 28 are driven to rotate, whereby the movable part 31 is advanced to push in the plunger 9 of the condensation vessel 3, and at the same time, the movable part 34 is retreated to pull the plunger 9 of the culture vessel 2, and accordingly, the cell liquid, in which the spheroids have been condensed, in the condensation vessel 3 is transferred to the culture vessel 2 by a predetermined amount. It is also possible that a simple container is used as the condensation vessel 3 at the lower side, and the spheroids are transferred from the culture vessel 2 to the container while the spheroids are being disrupted through filtration into small spheroids. That is, even with a device that includes only the first mechanism 27 of the inter-vessel cell liquid transfer device A, the filtration operation can be performed.

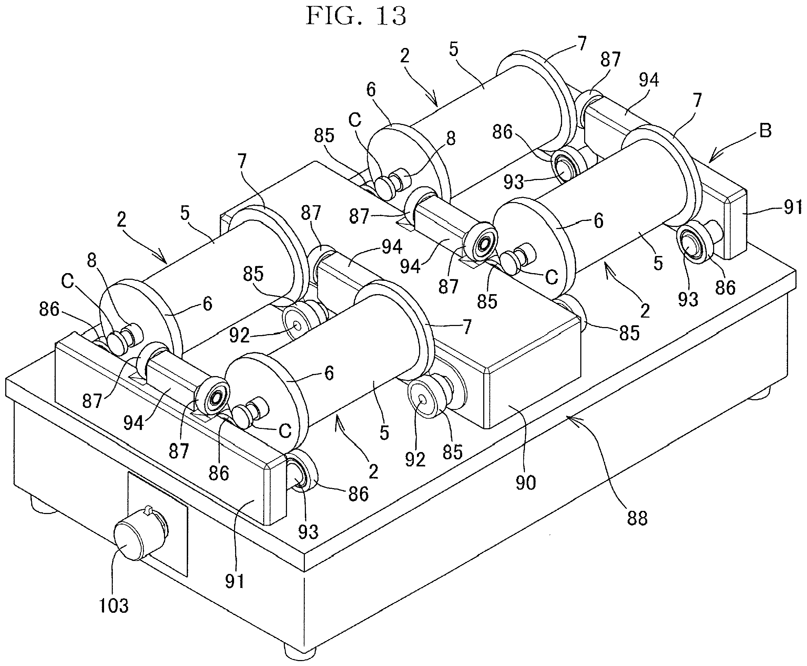

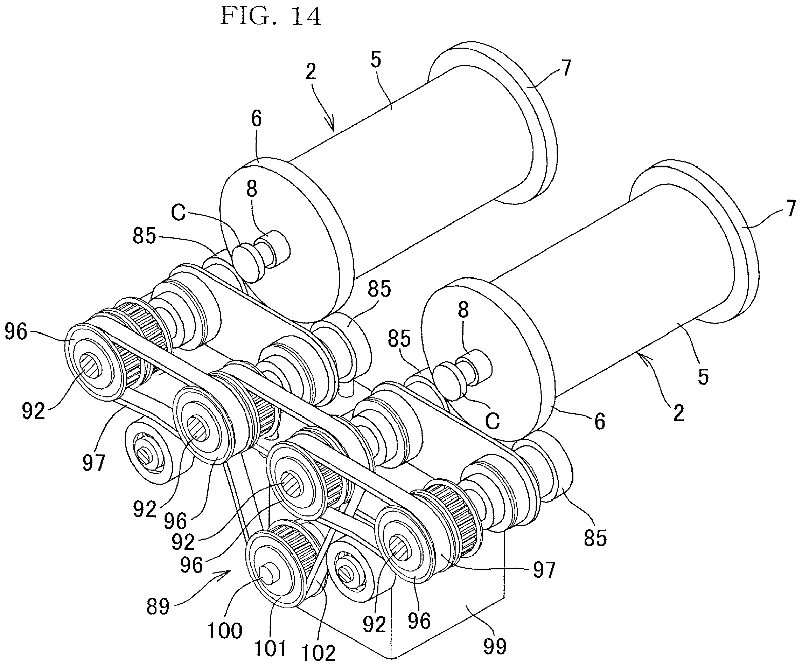

Finally, with reference to FIG. 13 and FIG. 14, the rotary cell culture device B is described. This rotary cell culture device B can simultaneously rotate a plurality of the culture vessels 2 in the state shown in FIG. 7C. The rotary cell culture device B of the present embodiment simultaneously rotates four culture vessels 2 at a predetermined speed such that cells can be cultured in a suspended state in a pseudo-microgravity environment. The rotary cell culture device B of the present invention is designed to be compact such that the rotary cell culture device B can be installed and used in an incubator having a temperature adjustment function.

In the present invention, a "pseudo-microgravity environment" means a microgravity (simulated microgravity) environment which is artificially created to simulate a microgravity environment as in outer space or the like. Such a pseudo-microgravity environment is realized by cancelling the gravity of earth by stress caused by rotation, for example. The rotary cell culture device B of the present invention is a device in which: the rotation speed of a culture vessel is controlled; the gravity, the buoyancy of spheroids in the culture vessel, and the force applied from the flow of the culture medium caused by rotation are balanced; a pseudo-microgravity environment having about 1/10 to 1/100 of the gravity of earth by time average is created; and accordingly, a state where the spheroids do not sediment but float in a certain area is realized.

In the rotary cell culture device B, lower portions of the front flange 6 and the back flange 7 of each culture vessel 2 are rotatably supported by a pair of driving rollers 85, 85 and a pair of driven rollers 86, 86, and the outer faces extending in the radial direction of the front flange 6 and the back flange 7 are stopped by, when coming into contact with, restriction rollers 87, 87, thereby being prevented from shifting in the axial direction. Each restriction roller 87 is a restriction part in the present invention.

Specifically, a center block 90 having a drive mechanism 89 built therein is provided so as to protrude at a center portion on the upper face of a base 88 having a control circuit incorporated therein; side blocks 91, 91 are each provided so as to protrude, while being separated from the center block 90 with a minimum interval for receiving the front flange 6 and the back flange 7 of the culture vessel 2; four pairs of the driving rollers 85, 85 are provided at both sides of the center block 90; and two pairs of the driven rollers 86, 86 are respectively provided at the side blocks 91, 91, at positions opposed to the driving roller 85, 85. The driving rollers 85 and the driven rollers 86 have rotation shafts 92, 93 which are horizontal and oriented in the same direction. The restriction rollers 87 are provided with horizontal rotation shafts 95 orthogonal to the rotation shafts 92, 93, at both ends of a protruding portion 94 which protrudes at a center portion on the upper face of each side block 91. Here, the driven rollers 86, 86 stably support one of the front flange 6 or the back flange 7, placed thereon, of the culture vessel 2, and freely rotate in association with rotation of the culture vessel 2. The restriction rollers 87 are driven-rotated only when the restriction rollers 87 come into contact with the outer faces extending in the radial direction of the front flange 6 and the back flange 7, and are for keeping the attitude such that the front flange 6 and the back flange 7 do not come off the driving rollers 85, 85 and the driven rollers 86, 86.

As shown in FIG. 14, the drive mechanism 89 is configured such that: pulleys 96 are respectively fixed at center portions of four rotation shafts 92 disposed in parallel; a belt, preferably a timing belt 97, is wound around the pulleys 96, 96 of each pair of the rotation shafts 92, 92; further, a belt, preferably a timing belt 102, is wound around pulleys 98, 98 fixed to two rotation shafts 92, 92 at the center portion and from different pairs and a pulley 101 fixed to a drive shaft 100 of a single drive motor 99; and all the driving rollers 85 rotate in the same direction. Since the driving rollers 85 are fixed to both ends of the four rotation shafts 92, the four culture vessels 2 can be rotated about the shafts by the single drive motor 99, and accordingly, the cells therein can be suspension cultured. A rotation speed adjustment knob 103 for adjusting the rotation speed of the drive motor 99 and also for serving as a power supply switch is provided at a side face of the base 88.

The drive mechanism 89 described above has extendability, and can also be configured such that a larger number of culture vessels 2 can be simultaneously subjected to rotary culture. If the dimensions of the front flange 6 and the back flange 7 are set to be common among culture vessels 2, even if the outer cylinder parts 5 have different diameters, i.e., different volumes, it is possible to perform rotary culture in the rotary cell culture device B. For a large-scale cell culture system as in the present invention, a large number of culture vessels 2 are used for performing subcultures, and accordingly, the number of rotary cell culture devices B used is increased, and thus, there is a need to employ inexpensive ones.

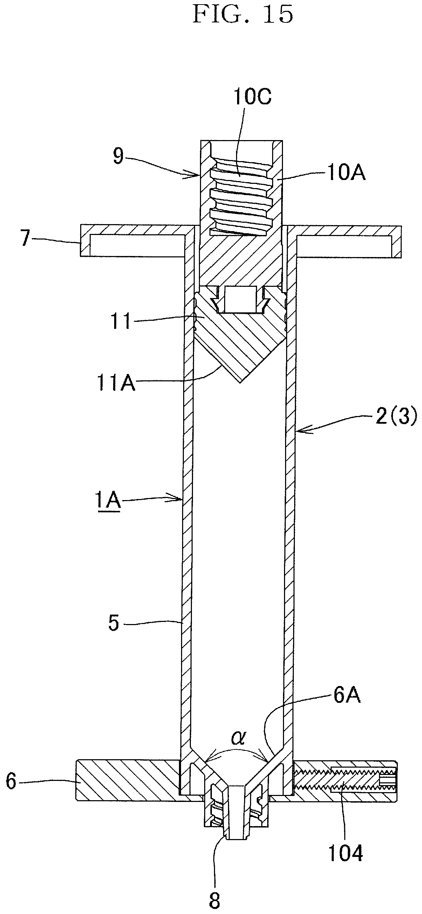

FIG. 15 shows a syringe 1A of another embodiment to be used as the culture vessel 2 or the condensation vessel 3. Similar to the description above, the syringe 1A is structured such that: the front flange 6 and the back flange 7 which have the same circular outer shape are provided integrally with both ends of the outer cylinder part 5 having a cylindrical shape; the head 8 provided at a center portion at an end portion of the outer cylinder part 5 at the front flange 6 side and serving as a discharge port has a standard Luer lock structure; the gasket 11 slidably and elastically fitted in the outer cylinder part 5 is provided at the distal end of a short shaft part 10A of the plunger 9; and the female screw 10C is provided at the other end portion of the short shaft part 10A. In the syringe 1 shown in FIG. 15, the inclination angle α of the inner face 6A is 90° in terms of a central angle.

In the syringe 1A, the outer cylinder part 5, the back flange 7, and the head 8 having the Luer lock structure are integrally molded from synthetic resin, and the front flange 6 is fastened and fixed by a screw 104 in the radial direction, at an end portion of the outer cylinder part 5. However, the front flange 6 may be formed as a synthetic resin molded article, and may be adhered, heat welded, or ultrasonic welded to the end portion of the outer cylinder part 5. When a mechanical fixing means such as the screw 104 is used, the front flange 6 may be made from metal through cutting. The syringe 1A shown in FIG. 15 has a volume of 10 ml, but a syringe 1A that has a volume of 50 ml basically has the same structure.

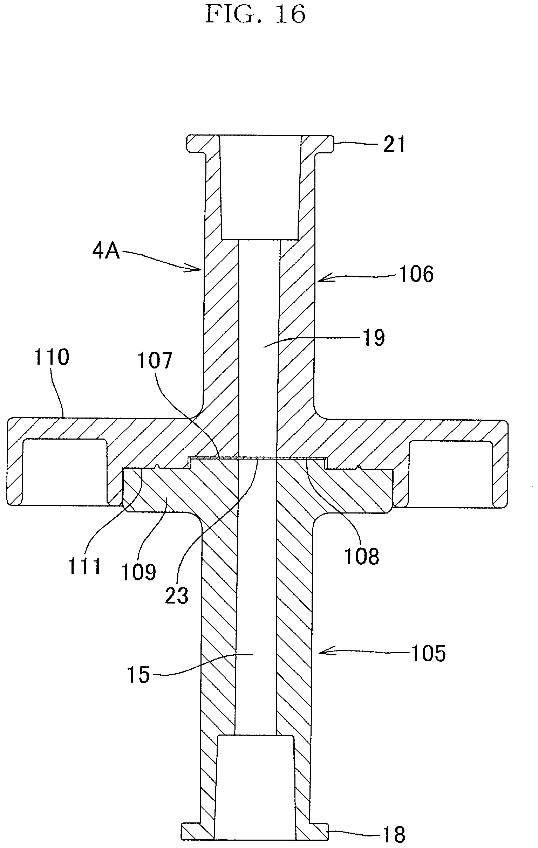

FIG. 16 shows a connection tool 4A of another embodiment. The connection tool 4 of the present embodiment is composed of a first member 105 and a second member 106. In the first member 105, the flow path 15 is provided at the center thereof, a support face 107 which holds the filter 23 is provided at one end side in the axial direction, and the connection part 18 to be connected to the head 8 is provided at the other end side in the axial direction. In the second member 106, the flow path 19 is provided at the center thereof, a support face 108 to be joined with the support face 107 of the first member 105 is provided at one end side in the axial direction, and the connection part 21 to be connected to the head 8 is provided at the other end side in the axial direction. A flange part 109 formed around the support face 107 of the first member 105 is fitted into a recessed portion 111 of a flange part 110 provided around the support face 108 of the second member 106. The filter 23 is interposed between the support face 107 of the first member 105 and the support face 108 of the second member 106, and the flange part 109 of the first member 105 and the flange part 110 of the second member 106 are integrated by means of ultrasonic welding. Here, the flow paths 15, 19 each have a diameter of 2 mm, and the filter 23 filters spheroids in an area also having a diameter of 2 mm. That is, in a state where the entirety of the effective surface of the filter 23 is covered by the spheroids, a differential pressure is given between the flow path 15 and the flow path 19, whereby passage of the culture medium can be suppressed to the minimum.

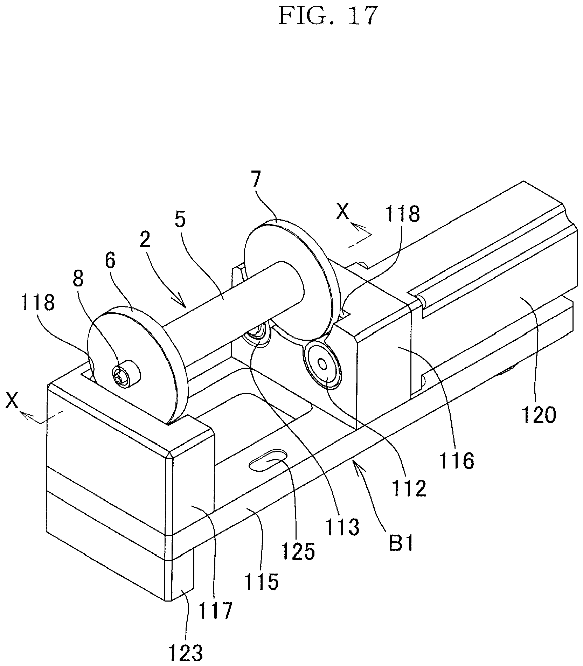

FIG. 17 shows a rotary cell culture device B1. The rotary cell culture device B1 of the present embodiment is a device that can rotate, at a predetermined rotation speed, one culture vessel 2 in an attitude with the shaft thereof horizontally oriented. Lower portions of the front flange 6 and the back flange 7 of the culture vessel 2 are each rotatably supported by a pair of rollers, and in the present embodiment, one side is supported by a pair of a driving roller 112 and a driven roller 113, and the other side is supported by a pair of driven rollers 114, 114. Specifically, a center block 116 is fixed at a center portion on the upper face of a base 115, a side block 117 is fixed to a side portion of the base 115, and recessed portions 118 provided at the opposed face sides of the respective blocks can receive lower portions of the front flange 6 and the back flange 7 of the culture vessel 2. In the center block 116, the driving roller 112 having a drive shaft 121 to which a drive motor 120, whose body is fixed to the center block 116, is also directly connected, and a driven roller 113 which freely rotates are provided in parallel at a bottom portion of the recessed portion 118. Meanwhile, in the side block 117, the driven rollers 114, 114 which freely rotate are provided in parallel at a bottom portion of the recessed portion 118.

Ball plungers 122, 122 for restricting displacement in the axial direction of the culture vessel 2 are embedded in standing wall portions of the recessed portions 118 of the center block 116 and the side block 117. Normally, a very small gap is provided between the ball plunger 122 and the outer face of the front flange 6 or the back flange 7, such that the culture vessel 2 can be rotated without any load. Each ball plunger 122 is a restriction part in the present invention. A fixation leg 123 is detachably provided at one side of the lower face of the base 115, and an adjuster 124 which can adjust the height in the up-down direction is detachably provided at the other side. Further, the base 115 has a level 125 built therein.

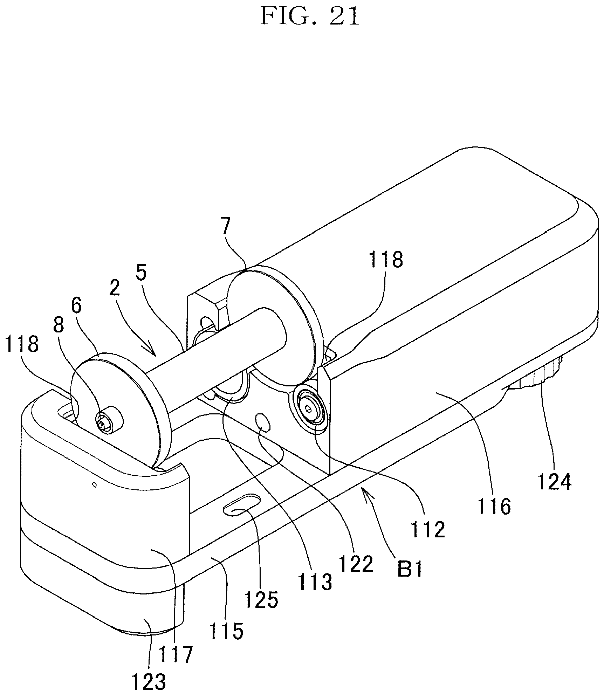

FIG. 21 shows an improved type of the rotary cell culture device B1, in which the center block 116 extends up to the other end of the base 115, a space for storing the drive motor 120 is provided therein, and the drive part is stored in a state sealed by the base 115 and the center block 116.

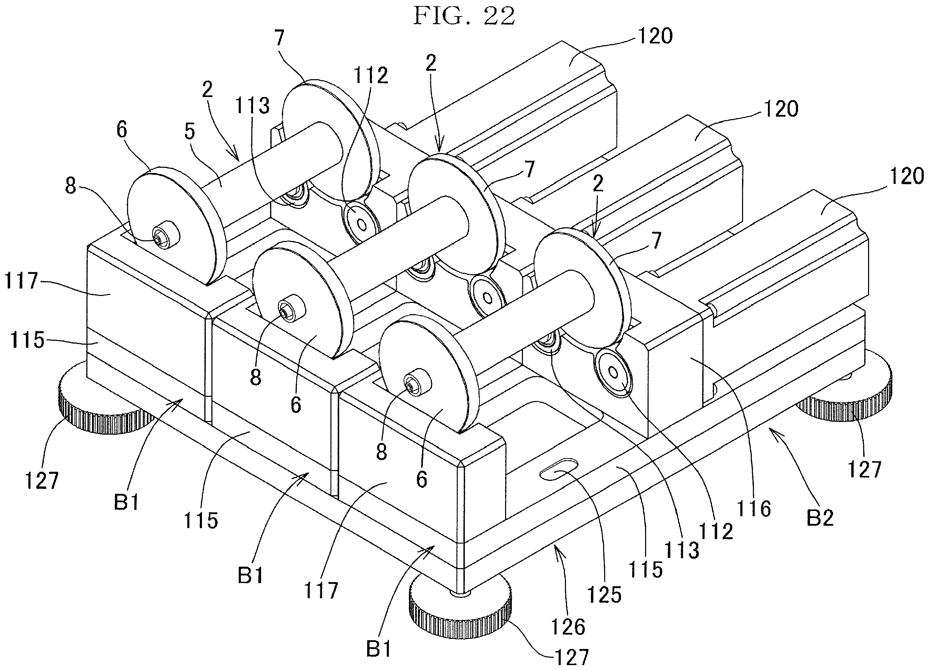

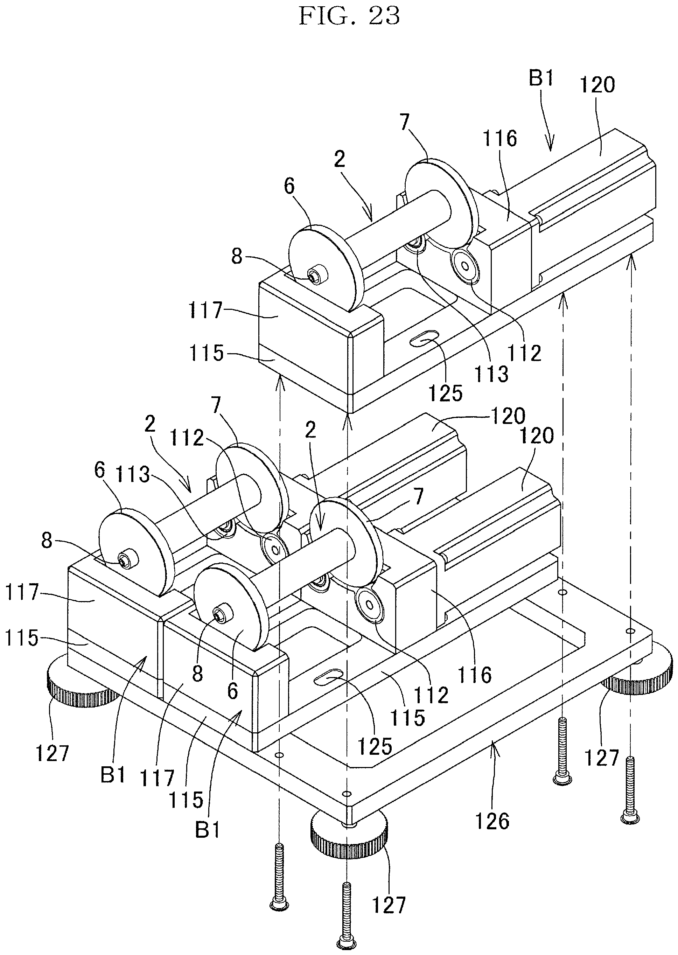

FIG. 22 and FIG. 23 each show a rotary cell culture device B2 having a structure in which three rotary cell culture devices B1 are arranged on and detachably mounted to a common base 126. In this case, the fixation leg 123 and the adjuster 124 of the rotary cell culture device B1 are removed, and the screw holes through which the fixation leg 123 and the adjuster 124 have been mounted are utilized to screw the rotary cell culture devices B1 onto the upper face of the base 126. In addition, adjusters 127 are attached to four corners of the base 126 so as to allow adjustment of the water level. For the adjusters 127, the adjusters 124 that have been removed from the rotary cell culture devices B1 may be used, and further, the fixation legs 123 may be used instead of some of the adjusters 127.

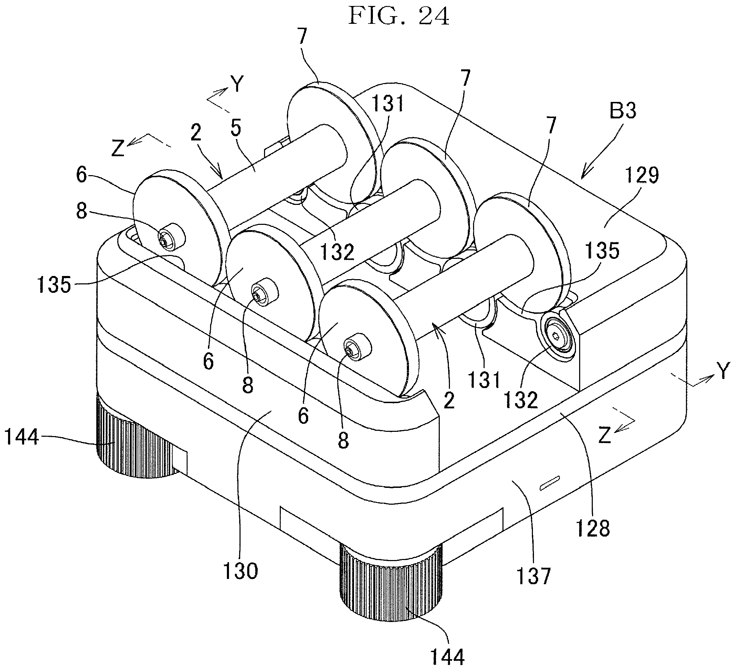

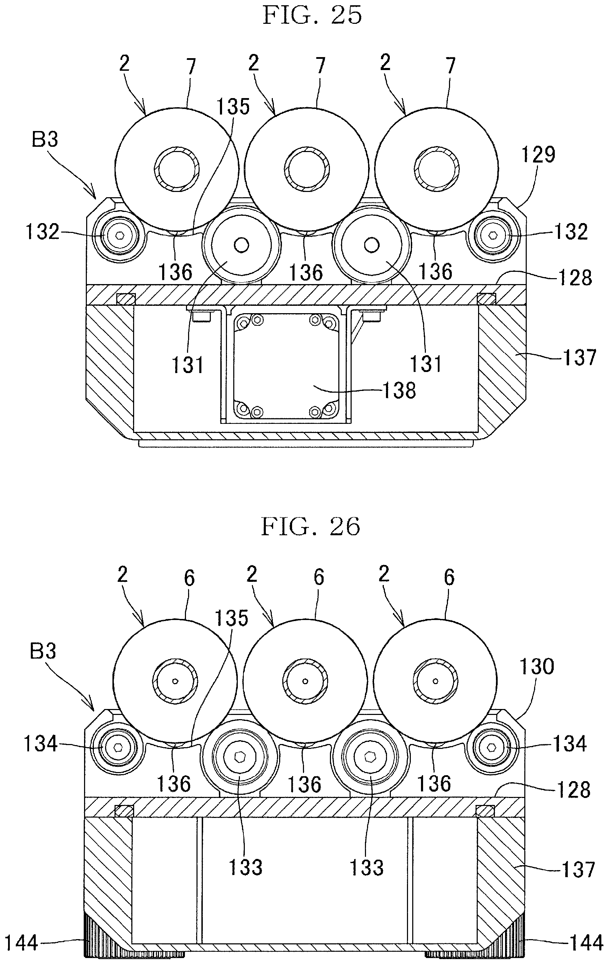

FIG. 24 to FIG. 27 each show a rotary cell culture device B3, which is a modification of the rotary cell culture device B, and in which three culture vessels 2 can be simultaneously subjected to rotary culture on the same culture condition. Basically, the rotary cell culture device B3 can be used in the same manner as that of the rotary cell culture device B2 described above. In the rotary cell culture device B3 of the present embodiment, a drive-side block 129 having a drive mechanism built therein and an opposed block 130 are fixed so as to be opposed to each other on the upper face of a base 128. In the drive-side block 129, two driving rollers 131, 131 are provided in parallel, driven rollers 132, 132 are provided in parallel at the outer side thereof, the back flange 7 of the culture vessel 2 at a center portion is placed on and supported by the two driving rollers 131, 131 at a center portion, and each of the back flanges 7 of the culture vessels 2 at the outer sides is placed on and supported by the driven roller 132 at the corresponding outer side and a corresponding one of the driving rollers 131. The two driving rollers 131, 131 are configured to rotate in the same direction in a synchronized manner. Meanwhile, in the opposed block 130, driven rollers 133, 133 having the same size are provided at positions opposed to the driving rollers 131, 131, and driven rollers 134, 134 having the same size are provided at positions opposed to the driving rollers 131, 131. Also in the present embodiment, recessed portions 135 similar to the recessed portions 118 are provided in the drive-side block 129 and the opposed block 130, respectively, and at the standing wall portions thereof, ball plungers 136 are provided so as to correspond to the flanges 6, 7 in order to limit displacement in the axial direction of the culture vessels 2.

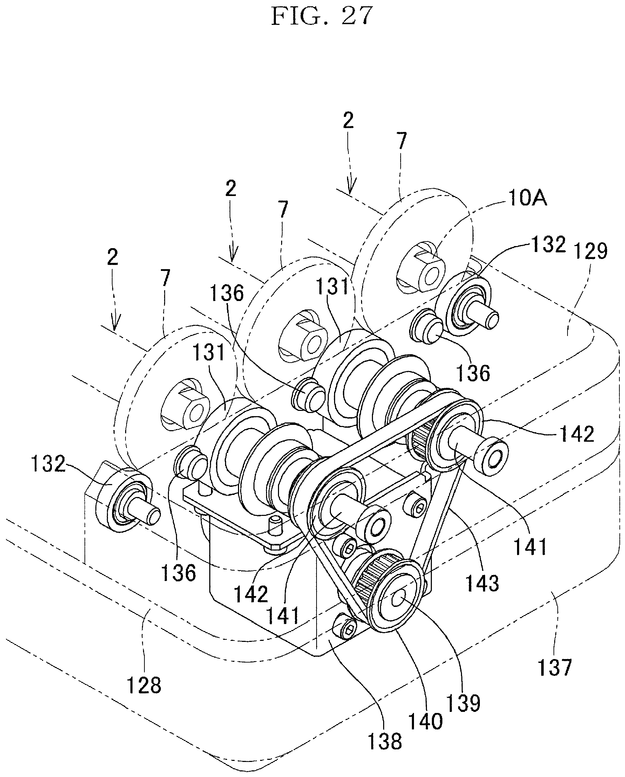

As shown in FIG. 27, the drive mechanism for the driving rollers 131, 131 is configured such that: a drive motor 138 is disposed in a box 137 provided below the base 128; a timing belt 143 is wound around a pulley 140 fixed to a drive shaft 139 of the drive motor 138 and pulleys 142 fixed to the rotation shafts 141 of the driving rollers 131, 131; and the driving rollers 131, 131 rotate in the same direction. In the box 137, a controller of the drive motor 138 is built therein to control the rotation speed of each culture vessel 2, and adjusters 144, 144 are provided at the lower end of the box 137 so as to allow horizontal adjustment.

### Example 1

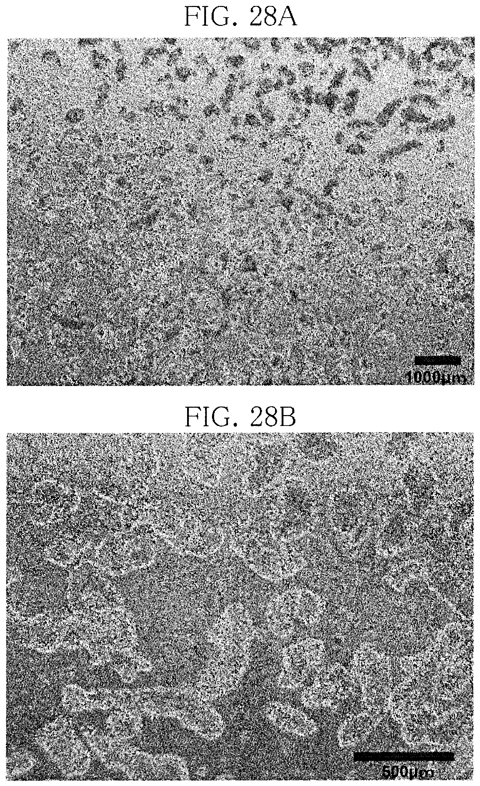

The effectiveness of the present invention was examined through experiments in the following procedure. First, a predetermined number of iPS cells (253G1) was put in a culture vessel having a volume of 50 ml, together with a culture medium (mTeSR1), and the resultant mixture was subjected to rotary culture in the rotary cell culture device B for 3 days. Then, by use of the inter-vessel cell liquid transfer device A, all globular spheroids were collected and transferred into a condensation vessel having a volume of 10 ml. Then, by use of the inter-vessel cell liquid transfer device A, the globular spheroids were caused to pass through a 70 µm filter from the condensation vessel to be disrupted into small spheroids, and the obtained small spheroids were transferred to a new 50 ml culture vessel. FIG. 28 shows phase contrast images of the small spheroids, wherein FIG. 28A shows a low magnification phase contrast image (the bar in the figure corresponds to 1000 µm), and FIG. 28B shows a high magnification phase contrast image (the bar in the figure corresponds to 500 µm). It is seen that the small spheroids having been subjected to filtration have slightly long shapes.

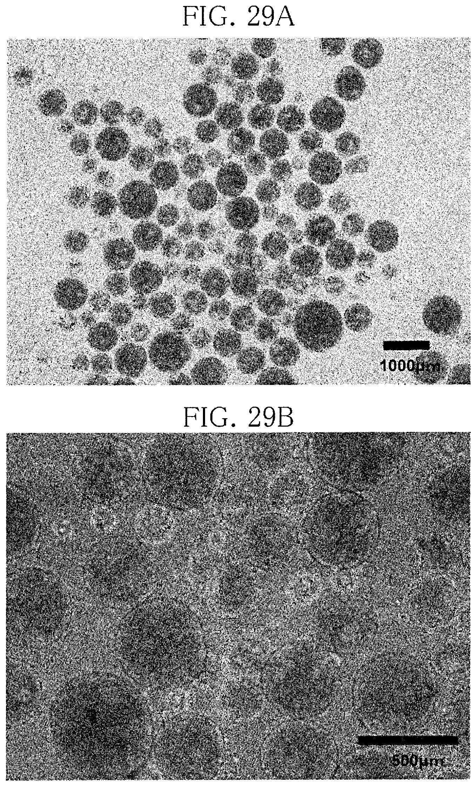

Next, the culture medium was added to the above-described small spheroids to dilute the mixture, and the resultant mixture was subjected to rotary culture for another 3 days. FIG. 29 shows phase contrast images of spheroids obtained by culturing the small spheroids, wherein FIG. 29A is a low magnification phase contrast image (the bar in the figure corresponds to 1000 µm), and FIG. 29B shows a high magnification phase contrast image (the bar in the figure corresponds to 500 µm). This result reveals that through the culture, the small spheroids proliferated into spherical spheroids and cells in the normal spheroid state were maintained, and a result similar to that obtained through manual filtering was able to be obtained also by the inter-vessel cell liquid transfer device A of the present invention.

In addition, it was found that, in the large-scale cell culture system of the present invention, the undifferentiated state was stably maintained up to the eighth subculture, and that the cells continued to proliferate at a proliferation rate of 3- to 5-fold. Although depending on the number of cells that are initially subjected to culture, it is seen that the number of cells in the order of 10⁹ to 10¹⁰ can be attained in large-scale culture through about six times of subculture at a proliferation rate of 3- to 5-fold. It has been confirmed that subculture can be carried out up to 10 times by use of the large-scale cell culture system according to the present invention.

In the Examples below, 253G1 cells and 409B2 cells were used as human induced pluripotent stem cells (hiPSCs). 253G1 cells (Oct3/4, Sox2, and Klf4 introduced; see Non-Patent Literature 2) were purchased under cell number HPS0002, and 409B2 cells (Oct3/4, Sox2, Klf4, L-Myc, Lin28, and p53 shRNA introduced; see Non-Patent Literature 4) were purchased under cell number HPS0076, from RIKEN BRC CELL BANK (Japan).

### Example 2

### <Production of spheroids from human induced pluripotent stem cells (hiPSC; 253G1 cells) through three-dimensional culture using syringe-type culture vessel>

### (1) Construction of globular spheroids

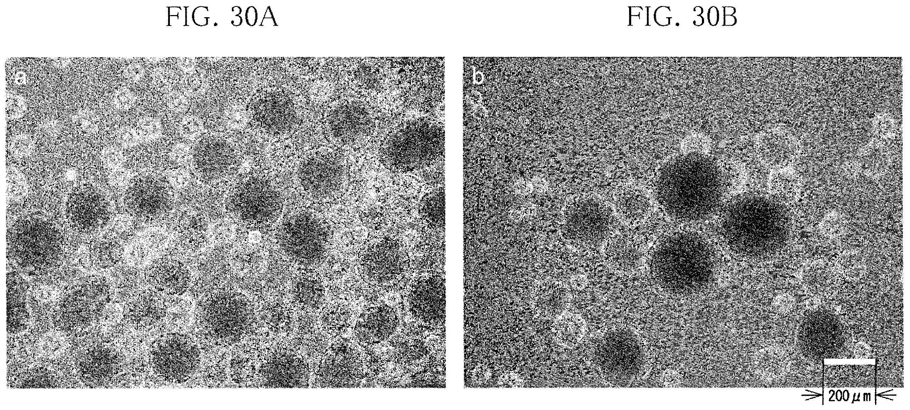

By use of a 6 cm- or 10 cm-culture dish coated with Matrigel (BD MatrigelTM, BD Biosciences), 253G1 cells were cultured in a human ES/iPS cell maintaining medium mTeSR1 (STEM CELL TECHNOLOGIES) or StemFit AK02N (Ajinomoto), the culture medium was replaced every other day, and subculture was maintained by use of a 5 mM EDTA. In order to perform three-dimensional culture, the 253G1 cells to be used in seeding were detached by use of a 5 mM EDTA to take the form of small spheroids (loose cell aggregates having a diameter of about 50 µm to 200 µm) composed of about 20 to 40 cells. The detached 253G1 cells of 5×10⁵ cells (small spheroids) were seeded into an mTeSR1 medium (10 ml) containing a ROCK (Rho-associated kinase) inhibitor Y27632 (WAKO Pure Chemicals, 10 µM) or a StemFit AK02N medium (10 ml) containing a ROCK inhibitor Y27632 in a syringe-type 10 ml vessel, and the resultant mixture was subjected to rotary culture at 6 rpm at 37°C for 3 days by use of the rotary cell culture device. After the culturing, phase contrast images of globular spheroids generated in the culture medium were taken by use of an inverted microscope EVOS (ThermoFisher) (see FIG. 30). FIG. 30 shows phase contrast images of spheroids obtained through the 3 days of rotary culture of the iPS cells (253G1 cells) using 10 ml vessels, wherein FIG. 30A is a phase contrast image of spheroids obtained through rotary culture in the mTeSR1 medium, and FIG. 30B is a phase contrast image of spheroids obtained through rotary culture in the AK02N medium. The white bar in the figure corresponds to 200 µm. About 50 globular spheroids were obtained, and the majority size thereof was 200 to 400 µm in diameter.

### Example 3

### <Production of spheroids from human induced pluripotent stem cells (hiPSC; 409B2 cells) through three-dimensional culture using syringe-type culture vessel>

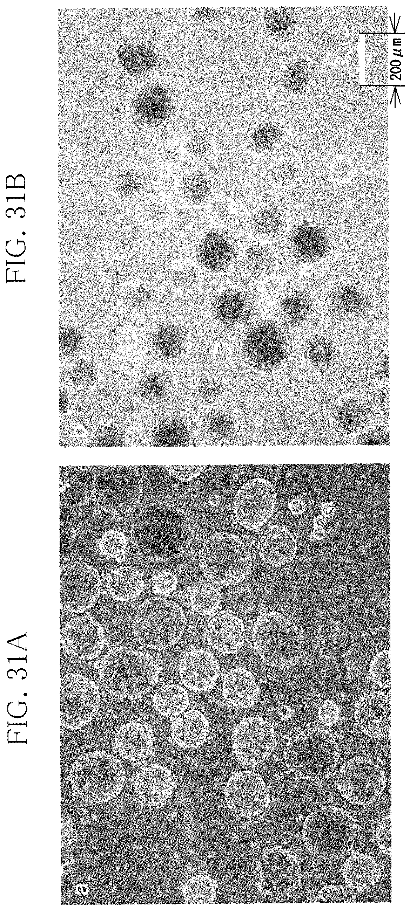

By use of a 6 cm- or 10 cm- culture dish coated with Matrigel (BD MatrigelTM, BD Biosciences), 409B2 cells were cultured in a human ES/iPS cell maintaining medium mTeSR1 (STEM CELL TECHNOLOGIES) or StemFit AK02N (Ajinomoto), the culture medium was replaced every other day, and subculture was maintained by use of a 5 mM EDTA. In order to perform three-dimensional culture, the 409B2 cells to be used in seeding were detached by use of a 5 mM EDTA to take the form of small spheroids (loose cell aggregates having a diameter of about 50 µm to 200 µm) composed of about 20 to 40 cells. The detached 409B2cells of 5×10⁵ cells (small spheroids) were seeded into an mTeSR1 medium (10 ml) containing a ROCK inhibitor Y27632 or a StemFit AK02N medium (10 ml) containing a ROCK inhibitor Y27632 in a syringe-type 10 ml vessel, and the resultant mixture was subjected to rotary culture at 6 rpm at 37°C for 3 days by use of the rotary cell culture device. After the culturing, phase contrast images of globular spheroids generated in the culture medium were taken by use of an inverted microscope EVOS (ThermoFisher) (see FIG. 31). FIG. 31 shows phase contrast images of spheroids obtained through the 3 days rotary culture of the iPS cells (409B2 cell) using 10 ml vessels, wherein FIG. 31A is a phase contrast image of spheroids obtained through rotary culture in the mTeSR1 medium, and FIG. 31B is a phase contrast image of spheroids obtained through rotary culture in the AK02N medium. The white bar in the figure corresponds to 200 µm. About 50 globular spheroids were obtained and the majority size thereof was 200 to 400 µm in diameter.

### Example 4

### <Serially passaged culture test>

In the present Example, a serially passaged culture test was performed. The procedure is as schematically shown in FIG. 5 to FIG. 7. That is, rotary culture was performed by use of a culture vessel, and between subcultures, filtration was performed by use of the inter-vessel cell liquid transfer device A, and at the same time, small spheroids were transferred into a new culture vessel.

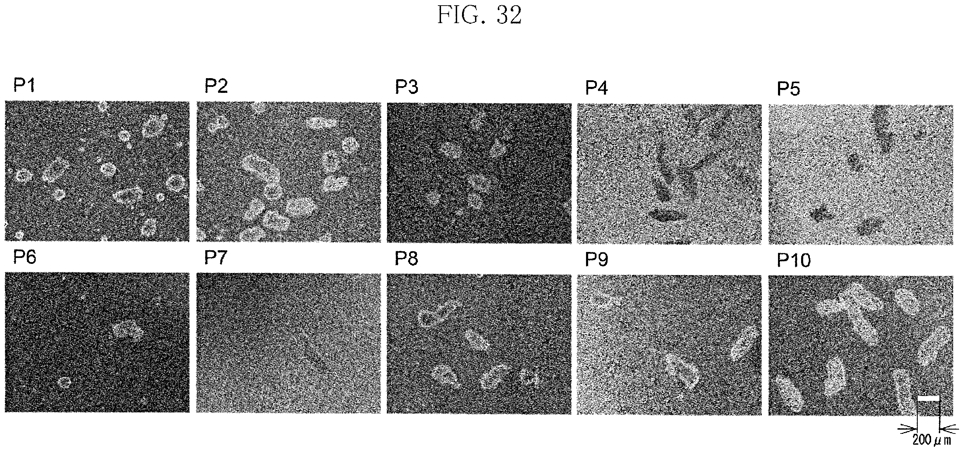

According to the method described in Example 2, 253G1 cells (5×105) detached by use of a 5 mM EDTA to take the form of small spheroids were seeded into a syringe-type 10 ml vessel, and the resultant mixture was subjected to rotary culture for 3 days by use of the rotary culture device in a StemFit AK02N medium containing 10 µM ROCK inhibitor Y27632, whereby globular spheroids were produced. Subsequently, the syringe-type 10 ml vessel used in the rotary culture was connected to a connection part having a filter built therein and a syringe-type 10 ml vessel for recovery, and the inter-vessel cell liquid transfer device was used. The globular spheroids were caused to pass through the filter, thereby being disrupted into small spheroids. A new medium was added to the obtained small spheroids to fill the vessel, and the resultant mixture was subjected to rotary culture at 6 rpm at 37°C for 4 days by use of the rotary cell culture device, whereby globular spheroids were produced. Then, disruption using the connection part having a filter built therein and the inter-vessel cell liquid transfer device, the medium filling, and culturing for 4 days were repeated 10 times (serially passaged culture). FIG. 32 shows phase contrast images of small spheroids observed immediately after disruption at each subculture. In each culture period, globular spheroids were able to be obtained every time. The majority diameter thereof was 200 to 400 µm, and some had a maximum diameter exceeding 700 µm. Small spheroids obtained at each disruption had similar bar shapes. The total number of culture days was 40, and no contamination occurred.

### INDUSTRIAL APPLICABILITY

The present invention can be used in realization of efficient, low cost, large-scale culture of pluripotent stem cells, in particular, iPS cells, or adhesive cells, to be used in regenerative medicine or the like.

### DESCRIPTION OF THE REFERENCE CHARACTERS

- A: inter-vessel cell liquid transfer device
- B, B1, B2, B3: rotary cell culture device
- C: cap
- M: culture medium
- S: spheroid
- SM: cell liquid
- 1: syringe
- 2: culture vessel
- 3: condensation vessel
- 4: connection tool
- 5: outer cylinder part
- 6: front flange
- 7: back flange
- 8: head
- 9: plunger
- 10: shaft part
- 10A: shaft part
- 10B: shaft part
- 10C: female screw
- 10D: male screw
- 10E: notch
- 11: gasket
- 12: plunger button
- 13: first member
- 14: second member
- 15: flow path
- 16: housing part
- 17: female screw part
- 18: connection part
- 19: flow path
- 20: male screw part
- 21: connection part
- 22: recessed portion
- 23: filter
- 24: packing
- 25: spacer
- 26: culture medium supply tube
- 27: first mechanism
- 28: second mechanism
- 29: base part
- 30: fixation part
- 31: movable part
- 32: drive mechanism part
- 33: fixation part
- 34: movable part
- 35: drive mechanism part
- 36: protection cover and the like
- 37: base plate
- 38: support plate
- 39: reinforcement plate
- 40: linear guide
- 41: linear actuator
- 42: guide rail
- 43: movable block
- 44: stage
- 45: drive motor
- 46: feed screw mechanism
- 46A: screw shaft
- 46B: nut member
- 47: linear guide
- 48: linear actuator
- 49: guide rail
- 50: movable block
- 51: stage
- 52: drive motor
- 53: feed screw mechanism
- 53A: screw shaft
- 53B: nut member
- 54: fixation stage
- 55: movable stage
- 56: interval adjuster
- 57: plunger holder
- 58: adjustment plate
- 59: pin
- 60: slit groove
- 61: long hole
- 62: fastening screw
- 63: handle
- 64: engagement recessed portion
- 65: plunger holder
- 66: adjustment plate
- 67: pin
- 68: slit groove
- 69: long hole
- 70: fastening screw
- 71: handle
- 72: engagement recessed portion
- 73: outer cylinder part holder
- 74: U-shaped recessed portion
- 75: flange fitting groove
- 76: holding member
- 77: hook
- 78: outer cylinder part holder
- 79: U-shaped recessed portion
- 80: flange fitting groove
- 81: holding member
- 82: hook
- 83: ground pad
- 84: ground pad
- 85: driving roller
- 86: driven roller
- 87: restriction roller (restriction part)
- 88: base
- 89: drive mechanism
- 90: center block
- 91: side block
- 92: rotation shaft
- 93: rotation shaft
- 94: protruding portion
- 95: rotation shaft
- 96: pulley
- 97: timing belt
- 98: pulley
- 99: drive motor
- 100: drive shaft
- 101: pulley
- 102: timing belt
- 103: rotation speed adjustment knob
- 104: screw
- 105: first member
- 106: second member
- 107: support face
- 108: support face
- 109: flange part
- 110: flange part
- 111: recessed portion
- 112: driving roller
- 113: driven roller
- 114: driven roller
- 115: base
- 116: center block
- 117: side block
- 118: recessed portion
- 120: drive motor
- 121: drive shaft
- 122: ball plunger (restriction part)
- 123: fixation leg
- 124: adjuster
- 125: level
- 126: base
- 127: adjuster
- 128: base
- 129: drive-side block
- 130: opposed block
- 131: driving roller
- 131: driving roller
- 132: driven roller
- 132: driven roller
- 133: driven roller
- 134: driven roller
- 135: recessed portion
- 136: ball plunger (restriction part)
- 137: box
- 138: drive motor
- 139: drive shaft
- 140: pulley
- 141: rotation shaft
- 142: pulley
- 143: timing belt
- 144: adjuster
- α: inclination angle

The following aspects are preferred embodiments of the invention.
1. A large-scale cell culture system for performing large-scale culture of pluripotent stem cells or adhesive cells by performing, in a closed system, subculture and transfer of spheroids and a culture medium by use of a vessel having a syringe structure, wherein
   the vessel comprises a front flange and a back flange which have a same circular outer shape and which are provided integrally with both ends of an outer cylinder part of the vessel, and
   the vessel allows rotary culture, utilizing the front flange and the back flange in a state where a head, of the vessel, serving as a port for a solution is closed by a detachable cap and a space, in the vessel, closed by a gasket of a plunger is filled with a cell liquid obtained by suspending cells in a culture medium.
2. The large-scale cell culture system according to aspect 1, wherein
   a shaft part of the plunger is able to be separated at a halfway point thereof.
3. The large-scale cell culture system according to aspect 1 or 2, wherein
   an inner face at a distal end side of the outer cylinder part is formed as a conically recessed portion, and an inclination angle α of the inner face is set in a range of 80° to 160° in terms of a central angle.
4. The large-scale cell culture system according to any one of aspects 1 to 3, wherein
   a process is performed between subcultures, the process including a cell condensation step of transferring spheroids obtained through culture, from a culture vessel to a condensation vessel, and
   in the cell condensation step,
   a head of the culture vessel and a head of the condensation vessel are coupled to each other through a connection tool,
   the coupled culture vessel and condensation vessel are attached to an inter-vessel cell liquid transfer device in a state where the head of the culture vessel is oriented downward, the inter-vessel cell liquid transfer device being capable of holding outer cylinder parts of the respective vessels and capable of performing driving so as to push and pull a plunger, and
   an entire amount of settled spheroids is transferred from the culture vessel to the condensation vessel.
5. The large-scale cell culture system according to aspect 4, wherein
   a volume of the condensation vessel is set to be smaller than a volume of the culture vessel.
6. The large-scale cell culture system according to aspect 4 or 5, wherein
   a process is performed between subcultures, the process including a dispensing-fining step of transferring a predetermined amount of the cell liquid, in which the spheroids have been condensed, in the condensation vessel into a plurality of new culture vessels, wherein
   in a state where the head of the condensation vessel holding the cell liquid in a condensed state and a head of a new culture vessel are connected to each other through the connection tool having a filter built therein, the condensation vessel and the new culture vessel are attached to the inter-vessel cell liquid transfer device,
   simultaneously with a plunger of the condensation vessel being pushed in, a plunger of the culture vessel is pulled, and
   a predetermined amount of the cell liquid, in which the spheroids have been condensed, in the condensation vessel is caused to pass through the filter to be transferred into the culture vessel, the filter having a function of disrupting spheroids into small spheroids.
7. The large-scale cell culture system according to aspect 6, wherein
   a process is performed between subcultures, the process including a dilution step of adding a new culture medium into the culture vessel into which the predetermined amount of the cell liquid, in which the spheroids have been condensed, has been transferred, wherein
   a culture medium is supplied into the culture vessel from a supply source of a new culture medium, the supply source being connected to the head of the culture vessel.
8. An inter-vessel cell liquid transfer device capable of transferring a solution from one vessel to another vessel, wherein
   the vessel comprises a front flange and a back flange which have a same circular outer shape and which are provided integrally with both ends of an outer cylinder part of the vessel,
   the vessel allows rotary culture, utilizing the front flange and the back flange in a state where a head, of the vessel, serving as a port for a solution is closed by a detachable cap and a space, in the vessel, closed by a gasket of a plunger is filled with a cell liquid obtained by suspending cells in a culture medium,
   the inter-vessel cell liquid transfer device comprises:
   a fixation part provided with an outer cylinder part holder which fixes outer cylinder parts of two respective vessels in a state where heads of the vessels are connected to each other through a connection tool;
   a movable part which moves a plunger holder which holds a plunger button provided at a distal end of a plunger; and
   a drive mechanism part which drives the movable part.
9. A rotary cell culture device capable of rotating, at a predetermined speed, a culture vessel filled with a cell liquid obtained by suspending cells in a culture medium, the rotary cell culture device capable of performing culture of cells in a suspended state in a pseudo-microgravity environment, wherein
   the vessel comprises a front flange and a back flange which have a same circular outer shape and which are provided integrally with both ends of an outer cylinder part of the vessel,
   in a state where a head, of the vessel, serving as a port of a solution is closed by a detachable cap and a space, in the vessel, closed by a gasket of a plunger is filled with the cell liquid, each of lower portions of the front flange and the back flange is rotatably supported by a pair of rollers, and at least one of the rollers that are in contact with the front flange or the back flange is a driving roller which is rotated by a drive motor, and an other of the rollers that are in contact with the front flange or the back flange is a driven roller, and
   the rotary cell culture device comprises a restriction part for keeping an attitude by contacting and stopping an outer face extending in a radial direction of the front flange and the back flange.
10. The rotary cell culture device according to aspect 9, wherein
   in order to enable simultaneous rotary culture of a plurality of the culture vessels, a plurality of sets of the driving roller, the driven roller, and the restriction part are provided on a base.

## Claims

1. A system, comprising
- vessels (2, 3), wherein the vessels (2, 3) each comprise an outer cylinder part (5), each of the vessels having a syringe (1) structure in which a head (8) of the vessel (2, 3), serving as a port for a solution is provided at a distal end of the outer cylinder part (5), wherein a space in the vessel (2, 3), closed by a gasket (11) of a plunger (9) is filled with a cell liquid (SM) obtained by suspending cells (S) in a culture medium (M);
- a connection tool (4) having a filter (23) built therein, wherein the filter (23) is configured to disrupt cell aggregates in the cell liquid (SM) into small cell aggregates as a result of the cell aggregates passing through the filter (23) when the cell liquid (SM) is transferred from one vessel (2, 3) to the other vessel (2, 3); and
- an inter-vessel cell liquid transfer device (A) capable of transferring the cell liquid (SM) from one vessel (2) to another vessel (3), wherein the inter-vessel cell liquid transfer device (A) is capable of holding the outer cylinder parts (5) of the vessels (2, 3) in a state where the heads (8) of the vessels (2, 3) are connected to each other through the connection tool (4) and capable of performing driving so as to push and pull the plunger (9) of one of the vessels (2,3).

2. System according to claim 1, wherein
each of the vessels (2, 3) includes an inner face (6A) that has a conically recessed shape.

3. System according to claim 2, wherein
an inclination angle α of the inner face (6A) is set in a range of 80° to 160° in terms of a central angle.

4. System according to any one of claims 1 to 3, wherein
the filter (23) has a filtration particle size of 40 to 100 µm.

5. System according to any one of claims 1 to 4, wherein
the gasket (11) is slidably and elastically fitted in the outer cylinder part (5) and provided at a distal end of a shaft part (10) of the plunger (9).

6. System according to any one of claims 1 to 5, wherein
the head (8) has a Luer lock structure.

7. System according to any one of claims 1 to 6, wherein
the inter-vessel cell liquid transfer device (A) is provided with a first mechanism (27) and a second mechanism (28), each being provided with a fixation part (30, 33), a movable part (31, 34), and a drive mechanism part (32, 35),
the fixation part (30, 33) is provided with an outer cylinder part holder (73, 78) which holds the outer cylinder part (5) of the respective vessel (2, 3),
the movable part (31, 34) is provided with a plunger holder (57, 65) holding a plunger button (12) provided at a proximal end of the plunger (9), and
the drive mechanism part (32, 35) drives the movable part (31, 34) to advance and retreat.

8. System according to claim 7, wherein
the first mechanism (27) and the second mechanism (28) are opposed to each other in the same vertical direction, and
the cell aggregates that settle in a conically recessed portion of one of the vessels (2) is transferred to the other one of the vessels (3) through the filter (23) of the connection tool (4).

9. System according to claim 8, wherein
a combination of cells and the cultivation medium (M), in which the cell aggregates settle in when the combination is left to stand still is used, and
the vessel (2) containing the cell liquid (SM) in which the cells (S) are suspended in the culture medium (M) is attached to the first mechanism (27) disposed in an upper side of the inter-vessel liquid transfer device (A) in a state where the head (8) is oriented downward, and then the movable part (31) of the first mechanism (27) is allowed to advance and the movable part (34) of the second mechanism (28) is allowed to retreat, so that the cell aggregates that settle in the conically recessed portion of the one of the vessels (2) is transferred to the other one of the vessels (3) attached to the second mechanism (28) through the filter (23) of the connection tool (4).
